(19) Europäisches Patentamt
European Patent Office
Office européen des brevets

(11) EP 4 182 936 B1

(12) EUROPEAN PATENT SPECIFICATION

(45) Date of publication and mention of the grant of the patent:
**29.04.2026 Bulletin 2026/18**

(21) Application number: **21746798.4**

(22) Date of filing: **15.07.2021**

(51) International Patent Classification (IPC):
***G16H 20/10*** (2018.01) ***G16H 50/20*** (2018.01)
***G16H 50/70*** (2018.01) ***G16H 70/40*** (2018.01)

(52) Cooperative Patent Classification (CPC):
**G16H 20/10; G16H 50/20; G16H 50/70; G16H 70/40**

(86) International application number:
**PCT/GB2021/051824**

(87) International publication number:
**WO 2022/013562 (20.01.2022 Gazette 2022/03)**

(54) **METHOD OF IDENTIFYING A DRUG FOR PATIENT-SPECIFIC TREATMENT**

VERFAHREN ZUR IDENTIFIZIERUNG EINES ARZNEIMITTELS ZUR PATIENTENSPEZIFISCHEN BEHANDLUNG

PROCÉDÉ D'IDENTIFICATION D'UN MÉDICAMENT POUR UN TRAITEMENT SPÉCIFIQUE AU PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.07.2020 GB 202010922**

(43) Date of publication of application:
**24.05.2023 Bulletin 2023/21**

(73) Proprietor: **Queen Mary University of London**
**London E1 4NS (GB)**

(72) Inventor: **CUTILLAS, Pedro Rodriguez**
**London EC1M 6BQ (GB)**

(74) Representative: **Lau, Sarah Jane et al**
**Kilburn & Strode LLP**
**Lacon London**
**84 Theobalds Road**
**London WC1X 8NL (GB)**

(56) References cited:
**WO-A1-2014/193982 US-A1- 2018 190 381**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 182 936 B1

## Description

### Field of the invention

The present invention relates to a computer-implemented method of identifying a drug with which to treat a patient. The present invention provides a ranking of drugs in order of predicted efficacy in a particular patient, such that a treatment choice can be made for that patient. The invention is particularly useful in the field of cancer therapy.

### Background to the invention

Cancers derived from the same tissue of origin and pathological classification exhibit high degrees of genetic and phenotypic variability across individuals. In practice, this heterogeneity translates in cancer patients having a wide range of responses to therapy. To address this issue, the field of personalized medicine aims to identify individualized therapeutic interventions by measuring the abundance of biomarkers indicative of the effectiveness of specific drug or drug combinations.

Current methods to stratify patients for therapy mainly rely on the association of genetic markers with drug responses, albeit protein markers are also employed; e.g., HER2 and estrogen receptor expression direct breast cancer therapies. Although current genetic indicators used in companion diagnostics can increase the proportion of patients who benefit from a given treatment, mutations and other genetic abnormalities are often inaccurate at stratification and provide results with a high frequency of false positives and negatives. Consequently, although patient selection using genetic biomarkers can increase the overall efficacy of a given therapy, these markers often show low accuracy in correctly stratifying individual patients for therapy. These observations may be explained by the complex biological landscape of cancer, where multiple biochemical pathways compensate each other and contribute to oncogenic phenotypes (Casado et al., 2013b; Klempner et al., 2013).

The application of machine learning (ML) to biomedicine promises to revolutionize how cancers are diagnosed and treated in the future. Projects such as the Cancer Target Discovery and Development (CTD2), DepMap, and Genomics of Drug Sensitivity in Cancer (GDSC) have evaluated ML as a means to predict drug responses by associating genomic features, gene expression patterns and copy number alterations to drug sensitivity. However, this approach has not been systematically applied using large scale proteomics and phosphoproteomics data, even though anecdotal evidence suggests that proteomic-derived features may be able to predict drug responses more accurately that genomic alternatives (Casado et al., 2013a; Casado et al., 2018; Frejno et al., 2017; Paulitschke et al., 2019; van Alphen et al., 2020). A limitation has been the low sample throughput of proteomics and phosphoproteomics by liquid chromatography coupled to tandem mass spectrometry (LC-MS/MS) compared to other -omics techniques. Most proteomics methods also involve comparing proteins after chemical or metabolic labeling, thus restricting the number of samples that can be directly compared and used as the input for ML model generation (Roumeliotis et al., 2017). Moreover, since labeling methods measure protein or phosphorylation sites as ratios, rather than providing absolute values of abundance, models of drug responses constructed with labelled proteomics data may be difficult to validate, and subsequently implement, in verification datasets and in the clinic. The advent of label-free and medium sample throughput proteomics techniques (Cutillas, 2017; Leutert et al., 2019; Montoya et al., 2011; Rudolph et al., 2016), together with the recent availability of systematic drug response profiles for a large number of cell lines and drugs (Basu et al., 2013; Smirnov et al., 2018; Yang et al., 2013), now allow the use of proteomics and phosphoproteomics data as the input of predictive models of drug responses. Thus, assessing the performance of ML models constructed using proteomics data as input is timely, and essential to evaluate the accuracy and the potential of proteomics to advance the field of precision medicine.

US 2018/190381 A1 discloses devices, systems, and methods in which multiple a priori known cell line genomics and drug-response data are used to build a large number of response (therapy outcome) predictors that are then tested with actual patient data in a statistically controlled manner to identify a drug for treatment of the patient.

### Summary of the invention

The invention is defined by the appended claims.

The present inventors have investigated an approach, named Drug Ranking Using ML (DRUML), for building and integrating ML models. DRUML uses ensembles of proteomic, phosphoproteomic and transcriptomic features to generate lists of ranked drugs based on their efficacy, for example in decreasing cancer cell proliferation. The ability of DRUML to predict drug rankings within a cancer cell population, without the need to compare to reference samples, is crucial for the clinical implementation of ML and fulfills a core aim of precision medicine.

Accordingly, in a first aspect the present invention provides a computer-implemented method of identifying a drug with which to treat a patient, the method comprising the steps of:

a. providing a dataset of expression values of biological markers from a sample taken from said patient;
b. using said dataset of expression values to calculate a plurality of drug response distance values $D_n$ for each of a plurality of drugs $d_n$, wherein *D* for each drug *d* is the difference between the distribution of expression of biological markers of sensitivity to drug *d* relative to the distribution of expression of biological markers of resistance to drug *d*;
c. providing one or more trained predictive models, wherein the one or more trained predictive models have been trained on a plurality of drug response distance values $D_n$ for at least the same plurality of drugs $d_n$ as in step b; and wherein the one or more trained predictive models have been trained to provide a ranking of the drugs from said plurality of drugs $d_n$ in order of their predicted efficacy in said sample taken from said patient;
d. inputting said plurality of drug response distance values $D_n$ obtained in step b. into said one or more trained predictive models; and
e. providing, using one or more of the trained predictive models, a ranking of the drugs from said plurality of drugs $d_n$ in order of their predicted efficacy in said patient.

## Detailed description of the invention

In a first aspect, the present invention relates to a computer-implemented method of identifying a drug with which to treat a patient. The method of the first aspect can alternatively be worded as a method of ranking drugs in order of their efficacy for treating a particular patient, in particular a computer-implemented method. The method involves calculating a plurality of drug response distance values $D_n$ for each of a plurality of drugs $d_n$, wherein *D* for each drug d is the difference between the distribution of expression of biological markers of sensitivity to drug *d* relative to the distribution of expression of biological markers of resistance to (the same) drug *d*.
The first step a. of the method of the invention is providing a dataset of expression values of biological markers from a sample taken from said patient.
Any large-scale -omic dataset can be used as the input of the method of the invention, which may be referred to herein as DRUML. The dataset of expression values used in the method of the first aspect of the invention may be obtained, for example, from phosphoproteomics, proteomics, or transcriptomics experiments and therefore may correspond to counts of phosphoproteins or phosphopeptides, proteins, peptides or gene transcripts. Transcriptomics data may be obtained, for example, using RNA-sequencing (RNA-seq). RNA-Seq data may be obtained from repositories such as the DepMap repository (Corsello et al., 2020).
For example, phospoproteomics data can be obtained using the inventor's TIQUAS (targeted and in-depth quantification of signalling) technique, as described in WO 2010/119261 (International patent application no. PCT/GB2010/000770) This technique allows for sensitive, rapid and comprehensive quantification of modified peptides. The method can, in one simple assay, simultaneously measure the amounts of thousands of phosphorylation sites and other modifications on proteins. Other computer programmes and workflows, such as MaxQuant [Nature Biotechnology 26, 1367 - 1372 (2008)] may be used to quantify peptides and these are compatible with the present invention. Other sources of publicly available proteomics and phosphoproteomics data include Jarnuczak et al., 2019 and Piersma et al., 2015).
The method of the invention may involve a step of obtaining a dataset of expression values of biological markers from a sample taken from said patient. This may be done using phosphoproteomics, proteomics, or transcriptomics techniques, as described herein. The step of taking a sample from the patient does not typically form part of the method.
The dataset of expression values of biological markers from a sample taken from said patient is then used in step b. to calculate a plurality of drug response distance values $D_n$ for each of a plurality of drugs $d_n$, wherein *D* for each drug *d* is the difference between the distribution of expression of biological markers of sensitivity to drug *d* relative to the distribution of expression of biological markers of resistance to drug *d*;
The *D* metric is essentially a measure of the distributions of sensitivity markers relative to resistance markers. *D* can be defined as the difference in overall expression of markers positively associated with drug sensitivity relative to markers positively correlated with drug resistance within a sample. The calculation of *D* involves analysing the expression of biological markers whose expression is increased in biological samples that are sensitive to drug *d* and the expression of biological markers whose expression is increased in biological samples that are resistant to the same drug *d*.
As used herein, "biological markers of sensitivity to drug *d*" means biological markers whose expression is consistently found to be increased in biological samples (typically cells) sensitive to drug *d* relative to their expression in biological samples (typically cells) resistant to drug *d*. As used herein "biological markers of resistance to drug *d*" means biological markers whose expression is consistently found to be increased in biological samples (typically cells) resistant to drug *d* relative to their expression in biological samples (typically cells) sensitive to drug *d*.
Such biological markers of sensitivity and resistance to a particular drug *d* are collectively referred to herein as empirical markers of drug responses (EMDRs). Such biological markers may be identified using a computer program such as the R package Limma (http://bioconductor.org/packages/release/bioc/html/limma.html), as described in the Examples herein. The biological markers of sensitivity and resistance are typically identified prior to carrying out the method of the first aspect of the invention, and are typically identified using a plurality of samples. The dataset of expression values of biological markers from a sample taken from the patient will typically include expression values for whichever biological markers that have previously been identified as biological markers of sensitivity and resistance to drug *d* are present in that sample.

The Examples herein describe the identification of various biological markers of sensitivity and biological markers of resistance to particular drugs, referred to in the Examples as EMDRs. Accordingly, in one embodiment the biological markers of sensitivity and/or biological markers of resistance may be any one or more of those identified in Figure 3. Figure 3C shows the most frequently identified phosphorylation sites, proteins and transcripts (see the charts headed "Phosphoproteomics", "Proteomics" and "Transcriptomics" respectively) as being biological markers of sensitivity or resistance, and the invention encompasses the use of any one or more of these biological markers.

The biological samples referred to in relation to the calculation of D may be cell lines, optionally cancer cell lines. Alternatively, such biological samples may be primary tissues, optionally cancer biopsies, which may be obtained directly from patients or from in vivo or ex vivo experiments. There may be some advantages to training the machine learning models using primary cells, such as primary cancer cells, isolated directly from tissues. This is because, in comparison to cell lines, primary cells are more similar in cell morphology and biological function to cells directly obtained from patients, since they have not been immortalised.

The present invention has the advantage that *D* values do not need comparison to other samples. Once EMDRs are identified these can be quantified within a sample and used to calculate *D* values within a biological sample (e.g. a tumour biopsy in a clinical assay where there are no other samples at hand to compare).

One exemplary method of calculating *D* is given in the Examples herein, and is described in relation to Figure 1A. In one embodiment, therefore, *D* is calculated for each drug *d* using:

$$D_d = [S^{Q2}-R^{Q2}]+[S^{Q3}-R^{Q3}],$$

where $S^{Q2}$ and $S^{Q3}$ are median and third quantile expression values of biological markers of sensitivity to drug *d*; and $R^{Q2}$ and $R^{Q3}$ are median and third quantile expression values of biological markers of resistance to drug *d.*

Any suitable alternative method may also be used to calculate *D*, including z-score, Kolmogorov-Smirnov and related methods, such as the following:

$$D_d = [\text{median } (M_S) + \text{Q3 } (M_S)] - [\text{median } (M_R) + \text{Q3 } (M_R)]$$

Where $M_S$ = expression of biological markers of sensitivity to drug *d* (proteins, phosphorylation sites, or RNA transcripts increased in biological samples (typically cells) sensitive to drug *d)*, and $M_R$ = expression of biological markers of resistance to drug *d* (proteins, phosphorylation sites or RNA transcripts increased in biological samples (typically cells) resistant to drug *d)*.

In terms of determining which biological samples (such as cell lines) are resistant or sensitive to a particular drug *d*, this can be done using any appropriate means. For example, the median area above the curve (AAC) cutoff can be used, as described in the Examples herein. Sensitivity data can alternatively be sourced using a database such as PharmacoDB (Smirnov et al., 2018).

The next step c. of the method of the invention involves providing one or more trained predictive models, wherein the one or more trained predictive models have been trained on the same plurality of drug response distance values $D_n$ as in step b.

The one or more trained predictive models (such as machine learning models) have been trained on a plurality of drug response distance values $D_n$ for a plurality of drugs $d_n$. For example, the models can be trained on a plurality of drug response distance values $D_n$ for at least the same plurality of drugs $d_n$ as in step b. The plurality of drugs $d_n$ can be any number of drugs, but the method of the invention is particularly useful to provide a ranking of drugs in order of their predicted efficacy in a particular patient when there are a large number of drugs from which to choose. For example, the plurality of drugs may consist of 100 to 500 drugs, for example 150 to 450, 200 to 400 or 300 to 350 drugs.

Typically, the plurality of drug response distance values $D_n$ comprises a number of the most positively correlated and most negatively correlated *D* values to the *D* value for drug *d*. For example, an equal or different number of positively correlated and negatively correlated *D* values to the *D* value for drug *d* may be used. In the Examples herein, the 7 most positively correlated and 7 most negatively correlated *D* values to the *D* value for drug *d* were used, however, any suitable number of the most positively correlated and most negatively correlated *D* values to the *D* value for drug *d* may be used, for example 1, 2, 3, 4, 5, 6, 7, 8, 9, 10, 11, 12, 13, 14, 15 or 20 each of the most positively correlated and most negatively correlated *D* values to the *D* value for drug *d*.

The trained predictive model may be a machine learning model or derived using statistical learning methods. Any suitable method may be used to train the predictive models. For example, the one or more trained predictive models may have been trained using a learning algorithm selected from random forest (rf), cubist, bayesian estimation of generalized linear models (bglm), partial least squares (pls), principal component regression (pcr), deep learning (dl) and neural network (nnet) learning algorithms. The present inventors found DL was the best performer using in-house training and validation datasets, while PCR and RF produced lower errors in verification datasets obtained from independent laboratories, as described in the Examples herein.

The one or more trained predictive models (such as machine learning models) have been trained to provide a ranking of the drugs from said plurality of drugs $d_n$ in order of their predicted efficacy in said sample taken from said patient. This allows for the prediction of the efficacy of the drugs in a patient, by providing a ranking of the drugs from said plurality of drugs $d_n$ in order of their predicted efficacy in the patient. In other words, the one or more trained predictive models have been trained to provide a ranking of the drugs from said plurality of drugs $d_n$ in order of their predicted efficacy in said patient. This means that the method of the invention can be used to inform treatment decisions for a particular patient. The ranking of the drugs provided by the one or more trained predictive models may, for example, take the form of a report that ranks the drugs in order of their predicted efficacy in the patient. As such, the output of the method of the first aspect of the invention may be a report that ranks the drugs in order of their predicted efficacy in the patient.

The method of the first aspect of the present invention is a method of identifying a drug with which to treat a patient. The method finds particular use in the identification of drugs with which to treat a patient who has been diagnosed with or is suspected of having cancer.

The cancer can be any cancer, for example lymphoma, leukemia (such as acute myeloid leukemia) or a solid tumour (such as oesophageal cancer or hepatocellular cancer). The sample taken from the patient is therefore typically a biopsy from a tumour.

Each drug *d* is therefore typically an anticancer drug (i.e. a drug specifically developed to treat cancer), or a different type of drug that has been repurposed for the treatment of cancer. Anticancer drugs include kinase inhibitors, for example an inhibitor of a human protein kinase selected from the group consisting of AGC kinases, for example protein kinase A (PKA), protein kinase B (PKB) (also known as Akt), protein kinase C (PKC) and protein kinase G (PKG); tyrosine kinases; tyrosine-kinase like kinases; calcium/calmodulin-dependent protein kinases; the casein kinase 1 group; CMGC group, for example CDK, MAPK, GSK3 and CLK kinases; and STE, the homologues of yeast Sterile 7, Sterile 11, and Sterile 20 kinases. Suitable kinase inhibitors for use in accordance with the invention include AZD-5438 (CDK2i;), GF-109203X (PKCαi; Tocris), PF-3758309 (PAKi; Calbiochem), Trametinib (MEKi; Selleckchem), MK-2206 (AKTi; Selleckchem), KU-0063794 (mTORi; Chemdea), TAK 715 (P38αi;), PKC-412 (PKC/Flt3i; Tocris), TBB (CK2i;), PF-3758309 (PAKi), and C4945 (CK2i; ). However, the DRUML method is not particularly limited to any particular type of anticancer drug and has indeed been shown to be effective in ranking drugs of diverse mode of action.

The method of the first aspect of the present invention may include an additional step f. of identifying a drug with which to treat a patient by selecting one of the highest-ranking drugs according to the ranking of the drugs from said plurality of drugs dn in order of their predicted efficacy. This may be the highest-ranking drug. However, in practice, the method of the first aspect will typically not be used in isolation to identify a drug with which to treat a patient. Typically, DRUML could assist drug prioritization by complementing information obtained from clinicopathological parameters and mutational analysis. Accordingly, the method may be used in conjunction with considering other factors for identifying a drug with which to treat a patient, for example cost, safety and/or regulatory issues (for example recommendations of NICE in the UK, EMA in EU and FDA in the USA). The ranking of the drugs using the method of the invention may therefore inform a treatment decision made either by a physician or using another computer-implemented method, for example a ML method. Accordingly, the drug selected for treating a patient may be the second highest-ranking drug or, for example, the third, fourth, fifth, sixth, seventh, eighth, ninth or tenth highest-ranking drug according to the ranking obtained by the method of the first aspect of the invention.

The present invention also extends to a method of training one or more predictive models to carry out the method of the first aspect of the invention.

Accordingly, in a second aspect the present invention provides a computer-implemented method of training one or more predictive models to provide a ranking of the drugs from a plurality of drugs $d_n$ in order of their predicted efficacy in a sample taken from a patient, the method comprising:

i. providing training data comprising a plurality of drug response distance values $D_n$ for each of a plurality of drugs $d_n$, wherein *D* for each drug *d* is the difference between the distribution of expression of biological markers of sensitivity to drug *d* relative to the distribution of expression of biological markers of resistance to drug *d*;
ii. training, using the training data, one or more predictive models to provide a ranking of the drugs from said plurality of drugs $d_n$ in order of their predicted efficacy in a sample taken from a patient.

Embodiments of the second aspect of the invention are as described above in relation to the first aspect of the invention.

As described above in relation to the first aspect of the invention, the one or more predictive models are trained to predict the efficacy of the drugs in a patient, by providing a ranking of the drugs from said plurality of drugs $d_n$ in order of their predicted efficacy in the patient. In other words, step ii. of the method of the second aspect of the invention involves training, using the training data, one or more predictive models to provide a ranking of the drugs from said plurality of drugs $d_n$ in order of their predicted efficacy in said patient.

The method of the second aspect of the invention may also be described as a method of providing a trained predictive model for use in relation to the first aspect of the invention. The method of the second aspect of the invention may be used to train a plurality of predictive models.

Accordingly, the method of the first aspect of the invention may involve the step of training one or more predictive models, as described in relation to the second aspect of the invention. In this embodiment, the method of the first aspect of the invention may be worded as follows:

a. providing a dataset of expression values of biological markers from a sample taken from said patient;
b. using said dataset of expression values to calculate a plurality of drug response distance values $D_n$ for each of a plurality of drugs $d_n$, wherein *D* for each drug *d* is the difference between the distribution of expression of biological markers of sensitivity to drug *d* relative to the distribution of expression of biological markers of resistance to drug *d*;
c. providing one or more trained predictive models, wherein the one or more trained predictive models have been trained using a method in accordance with the second aspect of the invention;
d. inputting said plurality of drug response distance values $D_n$ obtained in step b. into said one or more trained predictive models; and
e. providing, using one or more of the trained predictive models, a ranking of the drugs from said plurality of drugs $d_n$ in order of their predicted efficacy in said patient.

As described above in relation to the first aspect of the invention, the ranking of the drugs provided by the one or more trained predictive models may, for example, take the form of a report that ranks the drugs in order of their predicted efficacy in the patient. As such, the output of the method of the first aspect of the invention may be a report that ranks the drugs in order of their predicted efficacy in the patient.

In a third aspect, the present invention provides a computer-readable storage medium or media storing instructions for implementing a method of the first or second aspects of the invention.

In a fourth aspect, the present invention provides a system for identifying a drug with which to treat a patient, the system comprising a memory and one or more processors, wherein the one or more processors is configured to cause a method of the first aspect of the invention.

In a fifth aspect, the present invention provides a method of treating a patient having or suspected of having cancer, the method comprising identifying a drug with which to treat said patient according to a method of the first aspect of the invention, or using a system according to the third aspect of the invention, and administering said drug to said patient. In this aspect, the drug is an anticancer drug, as defined herein.

The method of treatment can be of a human or an animal subject and the invention extends equally to uses in both human and/or veterinary medicine. The drug is preferably administered to an individual in a "therapeutically effective amount", this being sufficient to show benefit to the individual and/or to ameliorate, eliminate or prevent one or more symptoms of a disease. As used herein, "treatment" includes any regime that can benefit a human or non-human animal, preferably a mammal, for example economically important mammals such as cattle, sheep, goats and pigs. The treatment may be in respect of an existing condition or may be prophylactic (preventative treatment).

The present inventors have designed an approach named Drug Ranking Using Machine Learning (DRUML) to rank drugs based on their predicted efficacy within a cancer model. In contrast to the inventors' previous "K-scores" approach described in PCT Application number PCT/EP2016/077845 (published as WO 2017/085116) which is limited to kinase inhibitors, DRUML predicts and ranks drug efficacy within a given cancer cell model. This is an important distinction because the ability to predict the best drug to treat a given patient, without the need to compare to other patients' samples, is important for the clinical implementation of ML in selecting the best therapy for given patients. This invention therefore fulfils a core aim of precision medicine.

The present invention also has the advantage over previously described approaches that it is not able just to predict whether a patient is sensitive or resistant to a particular drug, but it is able to rank the drugs according to predicted efficacy in a particular patient.

Preferred features for the second and subsequent aspects of the invention are as for the first aspect *mutatis mutandis.* It will be appreciated that all embodiments described herein are considered to be broadly applicable and combinable with any and all other consistent embodiments, as appropriate. Such combinations are considered to fall within the scope of the present invention.

The present invention will now be further described by way of reference to the following Examples which are present for the purposes of illustration only. In the Examples, reference is made to a number of Figures in which:

**Figure 1. Overview of Drug Ranking Using Machine Learning (DRUML)**

A) Drug response (AAC) values were modeled for 659 drugs with different DL/ML methods. Of these, 466 produced models by at least one learning algorithm. The input for DL/ML model generation are averaged values of empirical markers of drug responses (EMDRs), which are combined to derive a distance metric *D*. For each drug *d* and for each biological sample *b*, $D_{d,b} = [S^{Q2}-R^{Q2}]+[S^{Q3}-R^{Q3}]$, where $S^{Q2}$ and $S^{Q3}$ are median and third quantile expression values of empirical markers increased in cells sensitive to a given drug, respectively; and $R^{Q2}$ and $R^{Q3}$ are median and third quantile expression values of empirical markers increased in cells resistant to the same drug.

B) LC-MS/MS workflow for the generation of proteomic and phosphoproteomic datasets used to train DRUML.

C) Principal components analysis of proteomics, phosphoproteomics and drug response datasets of the named cell lines used to train DRUML.

D, E) Determination of empirical markers of drug sensitivity and resistance for barasertib. Cell lines are split into sensitive (dark grey) and resistant (light grey) groups based on barasertib response (AAC values) and limma used to identify response markers by resampling (E).

F) Distributions of empirical markers of resistance and sensitivity are shown for cell lines resistant (OCI-M1) and sensitive (P31-FUJ) to Barasertib or with intermediated phenotype (GDM-1). The median (Q2) and 3$^{rd}$ quantile (Q3) are marked for P31-FUJ barasertib's EMDR distributions.

G) Barasertib *D* values for the named cell lines calculated from the EMDR distributions shown in F.

**Figure 2. Dimensionality reduction using empirical markers of drug responses**

A, B, C) Associations between responses of cell lines to barasertib and the distance (*D_barasertib*) metric obtained from phosphoproteomics (A), proteomics (B) of RNA-seq (C) data. Distance values were computed by combining the expression of empirical markers of sensitivity and resistance as shown in Fig 1A and in the main text. *R* and p-values were generated by Pearson test.

D) Expression top 14 drug marker distances values which correlate both positively and negatively with drug sensitivity to barasertib. Rows are organized in order of Barasertib sensitivity (AAC). Dot color intensities and sizes are proportional to distance values normalized 0 to 1.

E) Overall correlation of each distance marker with barasertib sensitivity. Dot sizes are proportional to - log10 Spearman p-value.

**Figure 3. Overview of systematic empirical markers of responses to >400 drugs**

A) Number of empirical sensitivity and resistance markers identified per drug. Not all drugs were profiled in all cells cell lines; markers were successfully identified for 445 - 466 drugs with sufficient data points.

B) Frequency of phosphorylation sites (top), proteins (middle), and transcripts (bottom) being identified as empirical drug response markers.

C) The most frequent identified phosphorylation sites, protein and transcripts as being marker of sensitivity or resistance are shown.

D) Principal components analysis using empirical response markers as input. Representative drug classes are annotated.

**Figure 4. Performance of predictive models of responses to barasertib**
To illustrate DRUML for one drug, the performance of predictive models for barasertib using the D values from different datasets were compared.

A) Comparison of measured versus predicted responses retuned by the eight different learning methods using phosphoproteomics data as input. Solid, dashed and dotted lines signify 0%, 10% and 20% error boundaries, respectively.

B) As in (A) but D values were obtained from proteomics data.

C) Comparisons of standard errors (SE) in the validation set from the data in (A), (B) and Figure 11. P-values were calculated by Kruskal-Wallis test.

**Figure 5. Performance and accuracy of DRUML to rank drugs based on efficacy**

A) total number of models generated from each data input.

B) Training and validation errors for each model generated binned by ML method and input dataset.

C) Comparison between measured and predicted drug response values produced by DL analysis of phosphoproteomics distance values in the validation datasets. Each data-point represent a drug prediction with data point shape coded by the drugs' mode of action. Each cell line was analyzed in triplicate. Dotted line denotes slope or 1 with 0 intercept.

D) Absolute validation errors of learning models binned by ML method and input dataset.

**Figure 6. Accuracy assessment of DRUML to rank drugs based on efficacy using an independent phosphoproteomics dataset**

DRUML was used to predict drugs responses in the colorectal cancer (CRC) cell lines shown using phosphoproteomic data obtained from Piersma et al (Jimenez lab, PRIDE PXD001550).

A) Comparison of measured and predicted drug responses within cellular models. Each data-point represents a drug prediction.

B) Comparison of measured and predicted drug responses binned by developmental stage of the drug.

C) Distribution of validation absolute errors by ML model.

D, E) Number (D) and proportion (E) of accurate predictions within 0.05, 0.1, 0.15 and 0.25 absolute errors.

**Figure 7. Accuracy assessment of DRUML to rank drugs based on efficacy using independent proteomics datasets derived from 47 tumor models and 8 pathologies**
DRUML was used to predict drugs responses in the cell lines shown using proteomic data obtained from 12 different laboratories and compiled by Jarnuczak et al (Vizcaino lab, PRIDE PXD013455).

A) Comparison of measured versus predicted drug responses within cellular models using the random forest method. Each data-point represents a drug prediction.

B) Distribution of validation absolute errors in each tumor cell model from DRUML analysis using the random forest method.

C) Comparisons of the proportion of accurate predictions at 0.05, 0.1, 0.15 and 0.25 error cut-offs returned by the different ML methods.

**Figure 8. Qualitative assessment of proteomics and phosphoproteomics data**

A) Number of peptides, phosphopeptides and proteins identified from the proteomic analysis of 48 acute myeloid leukemia, esophageal and hepatocellular carcinoma cell lines shown in Figure 1.
B) Number of phosphopeptides (left) and unmodified proteins (right) quantified in each of the replicates from all the 48 analyzed cell lines.

**Figure 9. Number of empirical markers of drug responses (EMDRs) identified per drug**

Markers of resistance and sensitivity refer to phosphorylation sites, proteins or transcripts increased or decreased in cells resistant to a given drug, respectively.

**Figure 10. PCA based on empirical markers of drug response common to the different drugs identified from AML cell lines**

**Figure 11. Performance of barasertib response models using transcriptomic data**

**Figure 12. Comparison between measured and predicted drug response values produced by DL analysis of phosphoproteomics distance values in the training datasets**

**Figure 13. Qualitative assessment of phosphoproteomics data from 8 colorectal cancer cell lines obtained from Piersma et al.**

**Figure 14. Accuracy assessment of DRUML to rank drugs based on efficacy using independent proteomics datasets derived from 47 tumor models and 8 pathologies**

Results of DRUML-based drug response predictions using PCR (left) and DL (right) models are shown.

## Examples

### EXPERIMENTAL MODEL AND SUBJECT DETAILS

Cell lines were obtained from different repositories and cultured following the recommended cell culture conditions as provided from each source.

AML cell lines

The AML cell lines AML-193, CMK, K-052, Kasumi-1, KG-1, HEL, ME-1, ML-2, MOLM-13, MONO-MAC-6, MV4-11, OCI-AML2, OCI-AML3, OCI-AML5, P31/FUJ, PL-21, SIG-M5, SKM-1 and THP-1 were derived from male patients while, GMD-1, KMOE-2, HL-60, M-07e, NB-4 and NOMO-1 were derived from female patients. The sex of the patient from which OCI-M1 cells were derived is not specified in the DSMZ-German Collection of Microorganisms and Cell Cultures GmbH database.

Briefly, SIG-M5 and M-07e cell lines were maintained in IMDM supplemented with 20% FBS and 1% Penicillin/Streptomycin (P/S). M-07e cells were in addition supplemented with 10 ng/mL of GM-CSF. OCI-M1 and AML-193 cells were grown in IMDM supplemented with 10% FBS and 1% P/S. AML-193 cells were in addition supplemented with 5 ng/mL of GM-CSF. OCI-AML2, OCI-AML3 and OCI-AML5 cell lines were grown in α-MEM supplemented with 20% FBS and 1% P/S. OCI-AML5 cells were also supplemented with 5 ng/mL of GM-CSF. K-052 cells were maintained in α-MEM supplemented with 10% FBS and 1% P/S. GDM-1 and SKM-1 cells were grown in RPMI-1640 supplemented with 20% FBS and 1% P/S. SKM-1 cells were also supplemented with 1 ng/mL of GM-CSF. All other AML cell lines were maintained in RPMI-1640 supplemented with 10% heat inactivated FBS and 1% (RPMI/FBS medium). All cell lines were maintained at 37°C and 5% $CO_2$ in a humidified environment. Cell density was kept between 0.5 and $1.5x10^6$ cells per mL.

For proteomics and phosphoproteomics analysis, AML cell lines were seeded in IMDM medium supplemented with 10% FBS and 1% P/S in T75 flask ($20x10^6$ cells in 10 mL) and incubated for 3h at 37°C and 5% $CO_2$ in a humidified environment. Cell suspensions were then transferred to 15 mL falcon tubes and centrifuged at 1500 rpm for 5 min at 5°C. Supernatants were removed and cell pellets were washed twice with ice cold DPBS supplemented with phosphatase inhibitors (1 mM NaF, 1 mM $Na_3VO_4$). During washes, cells were centrifuged at 1500 rpm for 5 min at 5°C. Cell pellets were transferred into 1.5 mL protein LoBind tubes, snap frozen in dry ice and stored at -80°C. Biological independent replicates for each cell line (n=3) were performed in different dates.

Hepatic cancer cell lines

The hepatic cancer cell lines HEP 3B2.1-7, HEP G2, JHH2, JHH4, SK-HEP-1, SNU182, SNU-398, SNU-423, SNU-449 and SNU-475 were derived from male patients while the cell line SNU-387 was derived from a female patient. The sex of the patient from which PLC/PRF/5 cells are derived is not specified in the American Type Culture Collection (ATCC) database.

Cell lines SNU-387, SNU-423, SNU-182, SNU-398, SNU-475 and SNU-449 were maintained in RPMI-1640 supplemented with 1 mM sodium pyruvate, 10% FBS and 1% P/S. Cell lines HEP 3B2.1-7, HEP G2, JHH2, JHH4, PLC/PRF/5 were grown in MEM supplemented with 1 mM sodium pyruvate, 2 mM L-glutamine, 1X NEAA solution, 10% FBS and 1% P/S and the SK-HEP-1 cell line was maintained in MEM supplemented with 1 mM sodium pyruvate, 2 mM L-glutamine, 1X NEAA solution, 20% FBS and 1% P/S. All cell lines were maintained at 37°C and 5% $CO_2$ in a humidified environment. Cell density was kept between 0.2 and 0.4x$10^6$ cells per mL with 2 to 3 times per week medium renewal.

For proteomics and phosphoproteomics analysis, hepatic cell lines were seeded in petri dishes (between 0.3 and 3.74x$10^6$ cells in 20 mL) and maintained in an incubator for 3 to 8 days at 37°C and 5% $CO_2$ in a humidified environment, until cell confluence reached 80% approximately. Medium was replaced with fresh complete medium 1.5 hours prior to cell collection. Cells were subsequently washed three times with cold DPBS supplemented with 1 mM NaF and 1mM $Na_3VO_4$ and lysed with 500 μL of urea buffer (8M urea in 20 mM in HEPES, pH 8.0 supplemented with 1mM NaF, 1mM $Na_3VO_4$, 1mM $Na_4P_2O_7$ and 1 mM β-glycerophosphate). After cell collection with scrapers, lysates were snap frozen in protein LoBind tubes and stored at -80°C for further sample preparation.

Esophagus cancer cell lines

The Esophagus cancer cell lines KYSE-70, KYSE-140, KYSE-410, KYSE-450 and OE-19 were derived from male patients, while the cell lines COLO-680N, KYSE-150, KYSE-510, KYSE-520 and EO-33 were derived from female patients.

The KYSE-150 cell line was maintained in RPMI-1640 supplemented with 49% F12, 2% FBS and 1% P/S while KYSE-450 was grown in RPMI-1640 supplemented with 45% F12, 10% FBS and 1% P/S. All other esophagus cell lines were maintained in RPMI-1640 supplemented with 10% FBS and 1% P/S. All cell lines were maintained at 37°C and 5% $CO_2$ in a humidified environment. Cell density was kept between 0.1 and 0.25x$10^6$ cells per mL.

For proteomics and phosphoproteomics experiments, esophagus cell lines were seeded in petri dishes (between 1.5 and 3.5x$10^6$ cells in 10 mL) and maintained in an incubator overnight at 37°C and 5% $CO_2$ in a humidified environment. Then, cells were washed twice with cold DPBS supplemented with 1 mM NaF and 1mM $Na_3VO_4$ and lysed in 500 μL of urea buffer (8M urea in 20 mM in HEPES, pH 8.0 supplemented with 1mM NaF, 1mM $Na_3VO_4$, 1mM $Na_4P_2O_7$ and 1 mM β-glycerophosphate), snap frozen and stored at - 80°C until further processing.

**METHOD DETAILS**

Sample preparation for phosphoproteomics and proteomics analysis

Phosphoproteomics and proteomics analysis was carried out as previously described (Casado et al., 2018; Montoya et al., 2011; Wilkes and Cutillas, 2017). AML cell pellets were lysed in 320 μL of urea buffer (8 M urea in 20 mM HEPES, pH: 8.0, supplemented with 1 mM $Na_3VO_4$, 1 mM NaF, 1 mM $Na_4P_2O_7$ and 1 mM β-glycerophosphate). AML cell lysates were homogenised by sonication for 90 cycles (30 s on 30 s off) while thawed esophagus and hepatic cell lines were homogenised for 15 cycles (30s on & 40s off) in Diagenode Bioruptor® Plus and insoluble material was removed by centrifugation.

Proteins were quantified using BCA protein assay. Then, 300 μg of protein were subjected to cysteine reduction and alkylation using sequential incubation with 10 mM dithiothreitol (DDT) and 16.6 mM iodoacetamide (IAM) for 1 h and 30 min, respectively, at 25°C with agitation. Trypsin beads (50% slurry of TLCK-trypsin) were equilibrated with 3 washes with 20 mM HEPES (pH 8.0), the urea concentration in the protein suspensions was reduced to 2 M by the addition of 900 μL of 20 mM HEPES (pH 8.0), 100 μL of equilibrated trypsin beads were added and samples were incubated overnight at 37°C. Trypsin beads were removed by centrifugation (2000 xg at 5°C for 5 min) and samples were divided in 250 μg for phosphoproteomics analysis and 50 μg (200 μL) for proteomics analysis.

For phosphoproteomics analysis, peptide solutions were desalted using Oasis HLB cartridges (Waters) following the manufacturer's indications. Briefly, cartridges were set in a vacuum manifold device and the pressure was adjusted to 5 mmHg. Then, cartridges were conditioned with 1 mL acetonitrile (ACN) and equilibrated with 1.5 mL of wash solution (0.1% trifluoroacetic acid (TFA), 2% ACN). Peptides were loaded in the cartridges and washed twice with 1 mL of wash solution. Finally, peptides were eluted with 500 μL of glycolic acid buffer 1 (1 M glycolic acid, 5% TFA, 50% ACN). Enrichment of phosphorylated peptides was performed with $TiO_2$ Beads. Desalting eluents were normalized to 1 mL with glycolic acid buffer 2 (1 M glycolic acid, 5% TFA, 80% ACN) and incubated with 25 μl of $TiO_2$ buffer (500 mg $TiO_2$ beads in 500 μL of 1% TFA) for 5 min at room temperature. $TiO_2$ beads were packed by centrifugation into empty spin columns previously washed with ACN. $TiO_2$ beads were sequentially washed by centrifugation (1500 xg for 3 min) with 100 μL of glycolic acid buffer 2, ammonium acetate solution (100 mM ammonium acetate in 25% ACN) and three times with neutral solution (10% ACN). For phosphopeptide elution, spin tips were transferred to fresh tubes, 50 μL of elution solution 1 (5% $NH_4OH$, 7.5% ACN) were added and tips were centrifuged at 1500 xg for 3 min. The elution step was repeated a total of 4 times. Finally, samples were snap frozen, dried in a SpeedVac vacuum concentrator and phosphopeptide pellets were stored at -80°C.

For proteomics experiments, peptide solutions were desalted using C18+carbon top tips (Glygen Corporation).

The tips were conditioned twice with 200 μL of elution solution 2 (70/30 ACN/ H2O + 0.1% TFA) and equilibrated twice with 200 μL of wash solution. Sample were loaded into the top tips and washed twice with 200 μL of wash solution. For peptide elution, the tips were transferred to fresh tubes, and peptides were eluted three times with 250 μL of elution solution 2. In all desalting steps, tips were centrifuged at 1500 xg for 5 min at 5°C. Eluted peptide solutions were dried in a SpeedVac vacuum concentrator and peptide pellets were stored at -80 °C.

Mass spectrometry

Mass spectrometry for identification and quantification of proteins and phosphopeptides was carried out by LC-MS/MS as described before (Montoya et al., 2011; Wilkes and Cutillas, 2017). For phosphoproteomics analysis, peptide pellets were reconstituted in 13 μL of reconstitution buffer (20 fmol/μL enolase in 3% ACN, 0.1% TFA) and 5 μL were loaded onto an LC-MS/MS system. For proteomics analysis, peptide pellets were reconstituted in 10 μL of 0.1% TFA, 2 μL of this solution were further diluted in 18 μL of reconstitution buffer and 2 μL were injected into the LC-MS/MS system.

The LC-MS/MS platform consisted of a Dionex UltiMate 3000 RSLC coupled to Q Exactive™ Plus Orbitrap Mass Spectrometer (Thermo Fisher Scientific) through an EASY-Spray source. Mobile phases for the chromatographic separation of the peptides consisted of Solvent A (3% ACN; 0.1% FA) and Solvent B (99.9% ACN; 0.1% FA). Peptides were loaded in a μ-pre-column and separated in an analytical column using a gradient running from 3% to 23% B over 60 min (for phosphoproteomics) or 120 min (for proteomics). The UPLC system delivered a flow of 2 μL/min (loading) and 250 nL/min (gradient elution). The Q Exactive Plus operated a duty cycle of 2.1s. Thus, it acquired full scan survey spectra (m/z 375-1500) with a 70,000 FWHM resolution followed by data-dependent acquisition in which the 15 most intense ions were selected for HCD (higher energy collisional dissociation) and MS/MS scanning (200-2000 m/z) with a resolution of 17,500 FWHM. A dynamic exclusion period of 30s was enabled with m/z window of ±10 ppm.

Phosphopeptides and protein identification

Peptide identification from MS data was automated using Mascot Daemon 2.6.0 workflow in which Mascot Distiller v2.6.1.0 generated peak list files (MGFs) from RAW data and the Mascot search engine (v2.6) matched the MS/MS data stored in the MGF files to peptides using the SwissProt Database (SwissProt_2016Oct.fasta). Searches had a FDR of ~1% and allowed 2 trypsin missed cleavages, mass tolerance of ±10 ppm for the MS scans and ±25 mmu for the MS/MS scans, carbamidomethyl Cys as a fixed modification and PyroGlu on N-terminal Gln, oxidation of Met and phosphorylation on Ser, Thr, and Tyr as variable modifications (phosphorylation was only included for searches performed using phosphoproteomics data).

## QUANTIFICATION AND STATISTICAL ANALYSIS

Phosphopeptide and protein quantification

Identified peptides were quantified using a label free procedure based on extracted ion chromatograms (XICs). Missing data points were minimized by constructing XICs across all LC-MS/MS runs for all the peptides identified in at least one of the LC-MS/MS runs (Cutillas, 2017). XIC mass and retention time windows were ±7 ppm and ±2 min, respectively. Quantification of peptides was achieved by measuring the area under the peak of the XICs. Individual peptide intensity values in each sample were normalized to the sum of the intensity values of all the peptides quantified in that sample. Data points not quantified for a particular peptide were given a peptide intensity value equal to the minimum intensity value quantified in the sample divided by 10. For the phosphoproteomics experiments, we obtained a phosphorylation index (ppIndex) by summing the signals of all peptide ions containing the same modification site. For the proteomics experiment, protein intensity values were calculated by adding the intensities of all the peptides derived from a protein. Protein score values were expressed as the maximum Mascot protein score value obtained across samples.

Data source and processing

Sensitivity data was sourced from PharmacoDB (Smirnov et al., 2018). RNA-Seq data was obtained from the DepMap repository (Corsello et al., 2020). Proteomics and phosphoproteomics data was generated in-house for 26 AML, 10 esophagus and 12 HCC cell lines (see above) or obtained from (Jarnuczak et al., 2019; Piersma et al., 2015). Drug response, proteomics and phosphoproteomics datasets were sigmoid normalized and proteomics and phosphoproteomics data were further normalized by centering and scaling. RNA-seq data was obtained as quantile normalized values.

Empirical markers of sensitivity and resistance method

To reduce the dimensionality of the input datasets, we obtained empirical markers of drug responses (EMDR) using ensembles of statistical differences between cells resistant or sensitive to a given drug. For each drug, cell lines were separated into relative resistant or sensitive populations using the median drug response value (AAC) as cutoff. Resistant and sensitive populations were split into 10 groups using the createMultiFolds caret function (https://cran.r-project.org/package=caret). AAC values in each of the sensitive groups were compared to each the resistant groups, leading to 100 comparisons. Resistant and sensitive samples in the repeats with p-values < 0.05 between AAC values were used to generate markers. Linear models were generated and contrasts were computed for phosphorylation sites, proteins or transcripts across the sampled cell lines using the Limma package (http://bioconductor.org/packages/release/bioc/html/limma.html). The significance of these contrasts were assessed by empirical bayes statistics and p-values adjusted for multiple testing using the Benjamini-Hochberg method. EMDRs were defined as significant if a fold value $\pm$ 0.8 and p-value < 0.05 were achieved in at least 80% of the repeats. Those found to be increased in sensitive cells relative to resistant were regarded as markers of sensitivity while those increased in resistant cells were considered to be resistant markers.

DRUML method

Drug enrichment values were computed using custom in house scripts from the DRUMLR package. For each drug and for each cell line, distance (*D*) values were computed by subtracting the median and 3$^{rd}$ quartiles of resistance marker expression from the median and 3$^{rd}$ quartiles of sensitivity marker expression. *D* values for each drug were correlated with all drug response data by spearman ranking and top 14 correlated *D* values (7 positively and 7 negative correlating) were used as the input for machine learning models. Models were built using the caret (https://cran.r-project.org/package=caret) and h2o packages (https://cran.r-project.org/package=h2o). Data was separated into training and validation populations with a partition ratio of 0.8 using the createDataPartion function in caret. *D* values were then sigmoidal normalized before being used to build regression models using deep learning (DL), neural network (nnet), Bayesian estimation in generalized linear models (bglm), random forest, partial least squares (pls), principal components regression (pcr), support vector machine (svm) and cubist ML models. The h2o R package was used for DL model generation and the caret R package for all other models. Each model underwent hyperparameter tuning with 10-fold cross validation using RMSE as the loss function, and were subsequently validated using the validation data and verified using MS data from other laboratories. The code used for making and assessing the different learning models is provided in the DRUMLR package.

**Overview of approach**

DRUML consists of an ensemble of ML models trained on the responses of cells to >400 drugs, which allows these agents to be ranked based on their predicted efficacy within a sample **(Figure 1A).** In principle, any large-scale omic dataset can be used as the input of DRUML. While the use of gene copy number and RNA-seq for the generation of learning models is well documented, the utility and relative performance of large-scale proteomics and phosphoproteomics data is less well explored. Here we used phosphoproteomics and proteomics datasets obtained from 48 AML, esophagus and hepatocellular cancer cell lines as the input for DRUML to build models that may be applied to leukemia and solid tumors **(Figure 1B, 1C**). To reduce the impact of data noise on model performance, we first reduced the dimensionality of the omics datasets obtained as part of this investigation by obtaining empirical markers of drug responses (EMDRs, **Figure 1A),** which are used to compute an overall metric of drug response (which we named drug response distance, *D*). The *D* metric is the difference in overall expression of markers positively associated with drug sensitivity relative to markers positively correlated with drug resistance within a sample. *D* values were then used as input features of predictive models of drug responses, which were trained and tested with our dataset and verified using independent datasets from other laboratories (described below). This an important feature of DRUML for two reasons: firstly, the use of averaged marker values circumvents the problem of missing predictors when making predictions in verification or in future datasets because *D* can be computed using incomplete omic data; secondly, *D* is an internally normalized metric obtained by subtracting averaged signals from two sets of phosphosites, proteins or transcripts within a given sample; therefore, once the model is built, application of DRUML to predict drug responses in a new cancer-derived sample does not require comparison against a control or reference sample set.

**Input datasets**

To develop DRUML, we first analyzed the proteomes and phosphoproteomes of a panel of 26 AML, 10 esophageal and 12 hepatocellular carcinoma cell lines in triplicate (three independent cultures per cell line) by LC-MS/MS as in previous work (Casado et al., 2018; Hijazi et al., 2020) **(Figure 1B).** This analysis required 288 LC-MS/MS runs and produced a sufficiently large basal phosphoproteomics and proteomics dataset containing 22,804 phosphopeptides and 6,455 proteins, which generated 3,283,776 and 929,520 quantitative data-points, respectively **(Figure 8).** To our knowledge these are the largest sets of phosphoproteomics with matched proteomics data for AML, esophageal and hepatocellular carcinoma cell lines available to date.

Drug response data in the form of area above the curve (AAC values) were obtained from PharmacoDB (Smirnov et al., 2018) for the same cell lines for which we produced phospho- and proteomics data. AAC values were sigmoid normalized so that values ranged from 0 (no effect of drug) to 1 (maximum cell killing) within a given cell line. Drugs were filtered by interquartile range to ensure that there was a sufficient range of sensitivity in the dataset, thus reducing the number of profiled drugs from 659 to 466. For comparison we also used RNA-seq data obtained from the DepMap portal (Corsello et al., 2020) as the input of the models. **Figure 1C** shows that principal component analyses of the proteomics, phosphoproteomics and drug response data grouped cell lines by cancer type. Thus, to ensure that the models generated were interrogating the biological mechanisms of sensitivity without the influence of tumor type, we constructed separate DRUML models for solid and AML tumor samples as explained below.

**Dimensionality reduction**

To illustrate the approach that we used to reduce dimensionality, **Figure 1D-G** shows the determination of EMDRs for barasertib. For each drug, cell lines were split into those that are resistant or sensitive (using the median AAC cutoff) to such drug and compared repeated resampling **(Figure 1D**, **1E**). The R package Limma was then used to identity phosphorylation sites, proteins and transcripts frequently associated with drug responses across the resampling groups. Markers consistently found to be increased or decreased in sensitive cells by Limma are stored as EMDRs and provided in the DRUMLR package. We refer to markers increased in sensitive cells as sensitivity markers and those decreased as resistance markers. As outlined above, our approach then involves combining the identified EMDRs into a distance metric (*D*), which is essentially a measure of the distributions of sensitivity markers relative to resistance markers; *D* is formally defined in **Figure 1A. Figure 1F** shows the distribution of phosphorylation site markers associated to resistance and sensitivity to barasertib in cells resistant (OCI-M1), sensitive (P31-FUJ) or with intermediate response (GDM-1) to the drug. These distributions are then measured to derive *D* values, which correlate with drug responses across these three cell lines **(Figure 1G)** and across all models tested **(Figure 2A, B, C).** As expected, the correlation was statistically significant when markers derived from AML or solid tumors were applied to the respective tumor type but not when solid tumor derived markers were used to compare AML cell responses and vice-versa **(Figure 2A, B, C).**

To reduce dimensionality further, for each drug, DRUML selects the top 14 (7 positively and 7 negatively correlated) *D* values from phosphoproteomics, proteomics and transcriptomic EMDRs for all the 466 drugs. **Figure 2D** shows that barasertib response correlates with *D* values for several drugs. As a positive control for the approach, we found that baratsertib *D* values obtained from the three different marker datasets were consistently correlated with responses to this drug. AT7867 and CR1.31B *D* values also correlated with barasertib responses, whilst rigosertib, SL.0101.1, FK866 and FH535 *D* values were anti-correlated **(Figure 2E).** Thus, *D* values from different drugs and datasets consistently showed an association to barasertib responses, highlighting that *D* values reproducibility associate with drug responses irrespective of the omic datasets from which these were obtained.

Systematic application of this approach to 466 drugs in AML and solid cancer cell lines **(Figure 3A)** led to the identification of 1,232 and 1,139 phosphorylation sites, 542 and 480 proteins and 3,046 and 3,699 transcripts markers of responses for AML and solid models, respectively **(Figure 3B).** On average, each drug was annotated with 128 $\pm$ 37 (mean $\pm$ SD, range 53-278) and 97.6 $\pm$ 43 (15-269) phosphorylation sites markers of drug responses for AML and solid models respectively, and with 40-50 protein markers of resistance or sensitivity on average in solid and AML models (10 - 131 range) **(Figure 9).** The number of RNA transcripts associated to drug responses was greater because of the size of the input data. As **Figure 3C** illustrates, several of these phosphorylation sites, proteins and transcripts were found to be markers of responses for several drugs. Of interest, phosphorylation sites on FAM129B, SRRM2, lamin (LMNA) and on the mTOR substrate 4EBP1 were found to be sensitivity markers for > 200 drugs, whilst NPM1 (protein name nucleophosmin, NPM) phosphosites and the full protein were frequent markers of resistance **(Figure 3C).**

We also sought to explore the biological relevance of our EMDRs, and we hypothesized that these proteins and phosphorylation sites should group the drugs for which they are markers based on the compounds' known targets and modes of action. We observed that, in general, drugs that target common enzymes or with similar mode of action grouped together in PCA space **(Figure 3D** and **Figure 10).** For example, barasertib grouped with other AurB inhibitors and also with the microtubule destabilizers vinblatine and vinorelbine **(Figure 3D),** consistent with the role of AurB in microtubule stabilization (Haase et al., 2017). Similarly, EGFR inhibitors grouped together in PCA space and these separated from IGFR and IR inhibitors **(Figure 3D).** Another example is provided by ERK MAPK pathway inhibitors which grouped by their intended target in PCA space **(Figure 3D).** Thus, our markers of drug response group drugs by their mode of action, consistent with the notion that these markers are indicative of the biological mechanisms that determine responses to the profiled drugs.

**ML models of responses to barasertib**

We next generated learning models of drug responses using the 14 top correlated D values for a given drug obtained, as explained above for barasertib **(Figures 1 and 2).** Since we did not have prior knowledge of the learning methods that would be more appropriate to predict drug responses from proteomics datasets, we first assessed the performance of diverse ML methods based on random forest (rf), cubist, bayesian estimation of generalized linear models (bglm), partial least squares (pls), principal component regression (pcr), deep learning (dl) and neural network (nnet) learning algorithms. Although, as discussed above, our primary aim was to compare models constructed from D values obtained from phosphoproteomics and proteomics data, for benchmarking, we also constructed models using D values obtained from RNA-seq data as the input. The RNA-seq dataset was obtained from public resources (Corsello et al., 2020) making it difficult to directly compare the results obtained using RNA-seq with those obtained from our in-house generated proteomics and phosphoproteomics data. Therefore, in this study we do not derive conclusions on the relative performance of RNA-seq derived models. For model generation we split the data with 80:20 ratios (training and validation sets, which were different for each drug in order to produce splits having similar response value distributions in the two datasets) and regression models were trained on the normalized drug response (AAC) data by 10-fold cross validation using the root mean standard error (RMSE) metric as the loss function. DL/ML models were then evaluated on the validation set by comparing predicted versus actual responses using absolute error or standard error (SE) and RMSE (for individual data points and overall model performance, respectively).

Using barasertib as an initial example, we assessed the performance of the different models generated using *D* values derived from phosphoproteomic and proteomic datasets as predictors (shown in **Figure 2D). Figure 4** and **Figure 11** show that DL and NNET using D values from phosphoproteomics data produced the smaller validation errors with all the response values from all cell lines predicted with absolute errors < 0.2 AAC units **(Figure 4A, 4B, 4C).** In the DL model, 12 out of 12 validation data-points were predicted within < 0.1 AAC units from phosphoproteomics *D* data **(Figure 4A),** whereas the protein *D* data predicted 7 out of 12 data points within 0.1 AAC units **(Figure 4B).** Direct comparison of SE derived from the different ML methods **(Figure 4C)** confirmed that for barasertib, the DL model constructed using phospho D data produced the lowest validation errors.

**DRUML model ensembles to rank drug responses**

Next, we used the approach described above (using barasertib as an example) to systematically construct predictive models for 466 drugs (of which 412 could be modeled) using the phosphoproteomics, proteomics and RNA-seq distance D data obtained from AML and solid tumors as input. In total we constructed 17,064 learning models **(Figure 5A)** which weight 4.31 gigabytes of disk space. About the same number of models were created from phosphoproteomic and proteomics datasets **(Figure 5A).** As with the analysis of models of barasertib sensitivity, the DL algorithm produced the smaller validation errors for the proteomics and phosphoproteomic data derived from solid and AML tumor cancer types with mean SE < 0.1 in all cases **(Figure 5B).**

We then tested whether the ML models would allow ranking drugs within a cell line based on their predicted efficacy. **Figure 5C** shows the ranking of drugs in AML cells used for the validation of the DL algorithm (ranking of training data is shown in **Figure 12).** We observed a remarkably high correlation between the predicted and actual responses within cell models across drugs of diverse model of action. The mean absolute validation errors between predicted and actual responses were 0.04, 0.051 and 0.11 for DL models derived from phosphoproteomics, proteomics and RNA-seq datasets, respectively. Inspection of means of training (resampling) and validation errors for models derived from other algorithms **(Figure 5D),** confirmed that DL produced smaller errors for all the datasets. Thus our results suggest that DRUML may be used for accurately ranking drugs of diverse mode of action within tumors based on their predicted efficacy.

**Verification in independent datasets**

Ultimately, to be useful, predictive models of drug response should be able to accurately predict treatment outcome irrespective of the laboratory from which the data was obtained. Therefore, to verify DRUML using data collected by independent laboratories, we strived to test whether the models generated with our training datasets would predict drug responses from publicly available label-free proteomics and phosphoproteomics datasets generated by other groups. We downloaded label-free phosphoproteomics data from 8 colorectal cancer cell lines from Piersma et al from PRIDE [(Piersma et al., 2015), pride id: PXD001550], which we reprocessed using our mass spectrometry informatics pipeline (Cutillas, 2017; Hijazi et al., 2020) leading to the identification and label-free quantification of 12,197 phosphopeptides **(Figure 13).** This dataset was then used by DRUML to predict drug responses from the models previously generated using solid tumor's phosphoproteomics data for six of these cell lines (for which drug response data is available). **Figure 6** shows that there was a significant correlation between the DRUML-derived drug responses predictions and the actual responses for these six cell lines and that the predictions were accurate for drugs with diverse mode of action **(Figure 6A)** and developmental phase **(Figure 6B).** Average absolute errors were <0.15 AAC units for all DL/ML models tested **(Figure 6C).** In this datasets PCR and RF learning algorithms performed as well or better than DL with >80% of all responses being predicted with absolute errors < 0.15 AAC units **(Figure 6D, E**).

We also applied DRUML to predict drug responses in a panel of 47 cell lines derived from diverse solid tumor types. The input for this analysis was proteomics data obtained from Jarnuczak [(Jarnuczak et al., 2019), pride id: PXD013455], who compiled data from 11 separate studies. The DRUML models generated with our solid tumor proteomics training dataset were used to predict responses using iBAQ values provided by Jarnuczak et al without further processing (except for the averaging of iBAQ values of replicate cell line measurements). **Figure 7A** and **Figure 14** show that DRUML-predicted and actual drug responses were highly associated across the 47 cell lines irrespective of their assigned pathology. The median SE of prediction was below 0.1 for all cell lines **(Figure 7B)** and, as with the predictions from phosphoproteomics data, >80% of drug responses were predicted with absolute errors < 0.15 AAC units, and 95% of them with errors < 0.25 AAC units, with RF and PCR derived predictions being as accurate as those provided by DL and NNET models **(Figure 7C).** Overall, these data indicate that proteomics data, collected using routine LC-MS/MS, may be used as the input of DRUML to accurately predict and rank the efficacy of drugs of diverse mode of action in cancer cells derived from different pathologies.

**Discussion**

In this study we have illustrated the usefulness of proteomics and phosphoproteomics data as the input of DL/ML in order to rank drugs based on their predicted efficacy in reducing the proliferation of a given cancer cell population. Our work led to the development of DRUML, an ensemble of predictive models trained for 412 drugs with different mode of action and developmental stage. This work was possible because methods for systematic and relatively high-throughput label-free analysis of proteomes and phosphoproteomes are now emerging (Aasebo et al., 2020; Aebersold and Mann, 2016; Bodenmiller et al., 2010; Cutillas, 2017; Leutert et al., 2019; van Alphen et al., 2020; Vowinckel et al., 2018; Wilkes et al., 2015), and drug response data for a large number of compounds have been made public recently (Chiu et al., 2019; Corsello et al., 2020; Menden et al., 2019; Smirnov et al., 2018). However, since the use of large-scale LC-MS/MS proteomics data for DL/ML model generation has not been investigated systematically before, as part of DRUML development, we assessed the suitability of such large scale datasets as the input of predictive drug response models. Our initial evaluation showed that phosphoproteomics data consistently produced the lowest training and validation errors, although the difference between the accuracy of proteomics- and phosphoproteomics-based models was small. This is consistent with previous findings from our and other laboratories, which showed that phosphoproteomics and proteomics data reflect the mechanisms underpinning drug responses (Casado et al., 2013a; Casado et al., 2018; Frejno et al., 2017; Roumeliotis et al., 2017; van Alphen et al., 2020).

To limit the impact that noise and missing values in omics datasets may have in DL/ML model performance, and to make the approach practical, instead of individual phosphosites, proteins or transcripts, DRUML uses as input a distance metric (which we termed D) that measures the differences in distribution levels between sensitivity and resistance markers for a given drug. This feature contributes to DRUML robustness because *D* is an internally normalized value, it considers scores of markers (thus diluting the contribution of outliers) and addresses the potential issue of missing features in validation and verification datasets. Indeed, since each *D* value is calculated on average by hundreds of proteins, phosphorylation sites or transcripts **(Figure 3, Figure 9),** *D* metrics may be computed even when not all individual EMDRs are identified in the samples being analyzed. DRUML uses *D* values for the respective drug and those calculated for other drugs. For example, the *D* values chosen to build DL/ML models for barasertib include *D_barasertib* values and also *D* values for rigosertib, AT7867 and others **(Figure 2).** In our study, to avoid overfitting, we used the top 14 *D* values to construct DL/ML models for each drug. By controlling the number of *D* values to be included in models, it is possible to tune learning model performance.

The calculation of *D* values relies on measuring EMDR and we obtained >2000 phosphorylation sites and >800 proteins of such EMDR as input for DRUML **(Figure 3 and Figure 9).** Previous studies have suggested that, in general, drug efflux pump expression is the predominant variable impacting drug resistance for a variety of drugs (Roumeliotis et al., 2017) and that, in particular, kinase activation (detected as phosphorylation of kinase activity markers) underlies responses to kinase inhibitors (Casado et al., 2018; van Alphen et al., 2020). In our work we did not investigate the biochemical function of our set of predictive biomarkers as this was outside the scope of the present investigation. Further examination of the ontologies that these markers belong to may provide insights into biochemical pathways that mediate drug sensitivity or resistance to different therapies. We nevertheless found that our EMDR sets grouped drugs based on their mode of action when analyzed by unsupervised classification methods **(Figure 3),** thus suggesting that EMDRs, used by DRUML as input, reflect the biological mechanisms of responses to the different drugs.

Out of the different learning algorithms tested, we found that DL was the best performer using in-house training and validation datasets **(Figures 4 and 5),** while PCR and RF produced lower errors in verification datasets obtained from independent laboratories **(Figures 6 and 7).** This contrasts with finding from previous studies using transcriptomic data as input, which found that DL outperformed other ML methods (Sakellaropoulos et al., 2019). This difference may be explained by the fact that DL performance is only significantly greater than that provided by other ML methods when applied to large datasets (LeCun et al., 2015). Therefore, DL methods may be better suited to train predictive models from phosphoproteomics and proteomics as larger data sets become available.

Assessment of DRUML using external verification datasets from 53 cell lines analyzed by independent laboratories (Jarnuczak et al., 2019; Piersma et al., 2015) revealed that around 80% of the drugs could be ranked with absolute errors < 0.15 AAC units across all cancer types with 95% of them showing errors < 0.25 AAC units **(Figures 6 and 7).** This represents a remarkable and surprising accuracy given that DRUML was trained using esophageal and liver cancers, whereas the verification datasets consisted of data from cell lines derived from bone, brain, breast, cervix, colorectal, ovary and prostate cancers.

In summary, in this study we have assessed the accuracy of DRUML to produce list of drugs ranked by their predicted efficacy in reducing the proliferation of a given cancer cell population. We trained and validated the approach with the analysis of 48 cell lines profiled in our laboratory and verified it with a set of 53 cancer cell models profiled by twelve other groups. Our results indicate that DRUML ranks drugs of different mode of action based on their predicted efficacy across different cancer types with low error. Ultimately, DRUML could assist drug prioritization by complementing information obtained from clinicopathological parameters and mutational analysis.

**References**

Aasebo, E., Berven, F.S., Bartaula-Brevik, S., Stokowy, T., Hovland, R., Vaudel, M., Doskeland, S.O., McCormack, E., Batth, T.S., Olsen, J.V., et al. (2020). Proteome and Phosphoproteome Changes Associated with Prognosis in Acute Myeloid Leukemia. Cancers (Basel) 12.

Aebersold, R., and Mann, M. (2016). Mass-spectrometric exploration of proteome structure and function. Nature 537, 347-355.

Basu, A., Bodycombe, N.E., Cheah, J.H., Price, E.V., Liu, K., Schaefer, G.I., Ebright, R.Y., Stewart, M.L., Ito, D., Wang, S., et al. (2013). An interactive resource to identify cancer genetic and lineage dependencies targeted by small molecules. Cell 154, 1151-1161.

Bodenmiller, B., Wanka, S., Kraft, C., Urban, J., Campbell, D., Pedrioli, P.G., Gerrits, B., Picotti, P., Lam, H., Vitek, O., et al. (2010). Phosphoproteomic analysis reveals interconnected system-wide responses to perturbations of kinases and phosphatases in yeast. Science signaling 3, rs4.

Casado, P., Alcolea, M.P., Iorio, F., Rodriguez-Prados, J.C., Vanhaesebroeck, B., Saez-Rodriguez, J., Joel, S., and Cutillas, P.R. (2013a). Phosphoproteomics data classify hematological cancer cell lines according to tumor type and sensitivity to kinase inhibitors. Genome biology 14, R37.

Casado, P., Rodriguez-Prados, J.C., Cosulich, S.C., Guichard, S., Vanhaesebroeck, B., Joel, S., and Cutillas, P.R. (2013b). Kinase-substrate enrichment analysis provides insights into the heterogeneity of signaling pathway activation in leukemia cells. Sci Signal 6, rs6.

Casado, P., Wilkes, E.H., Miraki-Moud, F., Hadi, M.M., Rio-Machin, A., Rajeeve, V., Pike, R., Iqbal, S., Marfa, S., Lea, N., et al. (2018). Proteomic and genomic integration identifies kinase and differentiation determinants of kinase inhibitor sensitivity in leukemia cells. Leukemia 32, 1818-1822.

Chiu, Y.C., Chen, H.H., Zhang, T., Zhang, S., Gorthi, A., Wang, L.J., Huang, Y., and Chen, Y. (2019). Predicting drug response of tumors from integrated genomic profiles by deep neural networks. BMC Med Genomics 12, 18.

Corsello, S.M., Nagari, R.T., Spangler, R.D., Rossen, J., Kocak, M., Bryan, J.G., Humeidi, R., Peck, D., Wu, X., Tang, A.A., et al. (2020). Discovering the anticancer potential of non-oncology drugs by systematic viability profiling. Nature Cancer 1, 235-248.

Cutillas, P.R. (2017). Targeted In-Depth Quantification of Signaling Using Label-Free Mass Spectrometry. Methods Enzymol 585, 245-268.

Frejno, M., Zenezini Chiozzi, R., Wilhelm, M., Koch, H., Zheng, R., Klaeger, S., Ruprecht, B., Meng, C., Kramer, K., Jarzab, A., et al. (2017). Pharmacoproteomic characterisation of human colon and rectal cancer. Mol Syst Biol 13, 951.

Haase, J., Bonner, M.K., Halas, H., and Kelly, A.E. (2017). Distinct Roles of the Chromosomal Passenger Complex in the Detection of and Response to Errors in Kinetochore-Microtubule Attachment. Dev Cell 42, 640-654 e645.

Hijazi, M., Smith, R., Rajeeve, V., Bessant, C., and Cutillas, P.R. (2020). Reconstructing kinase network topologies from phosphoproteomics data reveals cancer-associated rewiring. Nat Biotechnol 38, 493-502.

Jarnuczak, A.F., Najgebauer, H., Barzine, M., Kundu, D.J., Ghavidel, F., Perez-Riverol, Y., Papatheodorou, I., Brazma, A., and Vizcaíno, J.A. (2019). An integrated landscape of protein expression in human cancer. bioRxiv, 665968.
Klempner, S.J., Myers, A.P., and Cantley, L.C. (2013). What a tangled web we weave: emerging resistance mechanisms to inhibition of the phosphoinositide 3-kinase pathway. Cancer Discov 3, 1345-1354.
LeCun, Y., Bengio, Y., and Hinton, G. (2015). Deep learning. Nature 521, 436-444.
Leutert, M., Rodriguez-Mias, R.A., Fukuda, N.K., and Villen, J. (2019). R2-P2 rapid-robotic phosphoproteomics enables multidimensional cell signaling studies. Mol Syst Biol 15, e9021.
Menden, M.P., Wang, D., Mason, M.J., Szalai, B., Bulusu, K.C., Guan, Y., Yu, T., Kang, J., Jeon, M., Wolfinger, R., et al. (2019). Community assessment to advance computational prediction of cancer drug combinations in a pharmacogenomic screen. Nat Commun 10, 2674.
Montoya, A., Beltran, L., Casado, P., Rodriguez-Prados, J.C., and Cutillas, P.R. (2011). Characterization of a TiO(2) enrichment method for label-free quantitative phosphoproteomics. Methods (San Diego, Calif) 54, 370-378.
Paulitschke, V., Eichhoff, O., Gerner, C., Paulitschke, P., Bileck, A., Mohr, T., Cheng, P.F., Leitner, A., Guenova, E., Saulite, I., et al. (2019). Proteomic identification of a marker signature for MAPKi resistance in melanoma. EMBO J 38, e95874.
Piersma, S.R., Knol, J.C., de Reus, I., Labots, M., Sampadi, B.K., Pham, T.V., Ishihama, Y., Verheul, H.M., and Jimenez, C.R. (2015). Feasibility of label-free phosphoproteomics and application to base-line signaling of colorectal cancer cell lines. Journal of proteomics 127, 247-258.
Roumeliotis, T.I., Williams, S.P., Goncalves, E., Alsinet, C., Del Castillo Velasco-Herrera, M., Aben, N., Ghavidel, F.Z., Michaut, M., Schubert, M., Price, S., et al. (2017). Genomic Determinants of Protein Abundance Variation in Colorectal Cancer Cells. Cell Rep 20, 2201-2214.
Rudolph, J.D., de Graauw, M., van de Water, B., Geiger, T., and Sharan, R. (2016). Elucidation of Signaling Pathways from Large-Scale Phosphoproteomic Data Using Protein Interaction Networks. Cell Syst 3, 585-593 e583.
Sakellaropoulos, T., Vougas, K., Narang, S., Koinis, F., Kotsinas, A., Polyzos, A., Moss, T.J., Piha-Paul, S., Zhou, H., Kardala, E., et al. (2019). A Deep Learning Framework for Predicting Response to Therapy in Cancer. Cell Rep 29, 3367-3373 e3364.
Smirnov, P., Kofia, V., Maru, A., Freeman, M., Ho, C., El-Hachem, N., Adam, G.A., Ba-Alawi, W., Safikhani, Z., and Haibe-Kains, B. (2018). PharmacoDB: an integrative database for mining in vitro anticancer drug screening studies. Nucleic Acids Res 46, D994-D1002.
van Alphen, C., Cloos, J., Beekhof, R., Cucchi, D.G.J., Piersma, S.R., Knol, J.C., Henneman, A.A., Pham, T.V., van Meerloo, J., Ossenkoppele, G.J., et al. (2020). Phosphotyrosine-based Phosphoproteomics for Target Identification and Drug Response Prediction in AML Cell Lines. Mol Cell Proteomics 19, 884-899. Vowinckel, J., Zelezniak, A., Bruderer, R., Mulleder, M., Reiter, L., and Ralser, M. (2018). Cost-effective generation of precise label-free quantitative proteomes in high-throughput by microLC and data-independent acquisition. Sci Rep 8, 4346.
Wilkes, E., and Cutillas, P.R. (2017). Label-Free Phosphoproteomic Approach for Kinase Signaling Analysis. Methods Mol Biol 1636, 199-217.
Wilkes, E.H., Terfve, C., Gribben, J.G., Saez-Rodriguez, J., and Cutillas, P.R. (2015). Empirical inference of circuitry and plasticity in a kinase signaling network. Proc Natl Acad Sci U S A 112, 7719-7724.
Yang, W., Soares, J., Greninger, P., Edelman, E.J., Lightfoot, H., Forbes, S., Bindal, N., Beare, D., Smith, J.A., Thompson, I.R., et al. (2013). Genomics of Drug Sensitivity in Cancer (GDSC): a resource for therapeutic biomarker discovery in cancer cells. Nucleic Acids Res 41, D955-961.

## Claims

**1.** A computer-implemented method of identifying a drug with which to treat a patient, the method comprising the steps of:

a. providing a dataset of expression values of biological markers from a sample taken from said patient;
b. using said dataset of expression values to calculate a plurality of drug response distance values $D_n$ for each of a plurality of drugs $d_n$, wherein *D* for each drug *d* is the difference between the distribution of expression of biological markers of sensitivity to drug *d* relative to the distribution of expression of biological markers of resistance to drug *d*;
c. providing one or more trained predictive models, wherein the one or more trained predictive models have been trained on a plurality of drug response distance values $D_n$ for at least the same plurality of drugs $d_n$ as in step b; and wherein the one or more trained predictive models have been trained to provide a ranking of the drugs from said plurality of drugs $d_n$ in order of their predicted efficacy in said sample taken from said patient;
d. inputting said plurality of drug response distance values $D_n$ obtained in step b. into said one or more trained predictive models; and

e. providing, using one or more of the trained predictive models, a ranking of the drugs from said plurality of drugs $d_n$ in order of their predicted efficacy in said patient.

**2.** The method of claim 1, further comprising:
f. identifying a drug with which to treat a patient by selecting one of the highest-ranking drugs, wherein the selected drug is the highest, second-highest, third-highest, fourth-highest, fifth-highest, sixth-highest, seventh-highest, eighth-highest, ninth-highest or tenth-highest-ranking drug.

**3.** The method of claim 1 or 2, wherein *D* is calculated for each drug *d* using:

$$D_d = [S^{Q2}-R^{Q2}]+[S^{Q3}-R^{Q3}],$$

where $S^{Q2}$ and $S^{Q3}$ are median and third quantile expression values of biological markers of sensitivity to drug *d*; and $R^{Q2}$ and $R^{Q3}$ are median and third quantile expression values of biological markers of resistance to drug d.

**4.** The method of any one of claims 1 to 3, wherein said plurality of drug response distance values $D_n$ comprises the most positively correlated and most negatively correlated *D* values to the *D* value for drug *d*.

**5.** The method of claim 4, wherein said plurality of drug response distance values $D_n$ comprises an equal number of positively correlated and negatively correlated *D* values to the *D* value for drug *d*, optionally wherein said plurality of drug response distance values $D_n$ comprises the 7 most positively correlated and 7 most negatively correlated *D* values to the *D* value for drug *d*.

**6.** The method of any one of the preceding claims, wherein said biological markers of sensitivity to drug *d* are biological markers whose expression is consistently found to be increased in biological samples sensitive to drug *d* relative to their expression in biological samples resistant to drug *d*, and wherein said biological markers of resistance to drug *d* are biological markers whose expression is consistently found to be increased in biological samples resistant to drug d relative to their expression in biological samples sensitive to drug *d*, optionally wherein the biological samples are:

(i) cell lines, optionally cancer cell lines; or
(ii) primary cells obtained from patients, optionally primary cancer cells obtained from patients.

**7.** The method of any one of the preceding claims, wherein said biological markers of sensitivity to drug *d* and/or said biological markers of resistance to drug *d* are selected from the group consisting of: FAM129B(S691); SRSF6(S301); HSP90AA1(S252); SRRM2(S2102);SRRM2(T2104); DBNL(K288);DBNL(T291); EIF4EBP1(S83);EIF4EBP1(S85); EIF4EBP1(S44); LMNA(S392); TRA2A(S262);TRA2B(S266); TRA2A(S260);TRA2B(S264); LMNA(S390); ACIN1(S1004); BCLAF1(S512); MCM2(T25); HSP90AB1(S255);HSP90AB2P(S177); HSP90AB1(S255); SRRM2(M1168);SRRM2(T1177); IRF2BP2(S175); ADD1(S726);ADD2(S713);ADD3(S693); FAM129B(S691);FAM129B(S692); EIF4EBP1(Y34);EIF4EBP1(T37); EIF4EBP1(T41); EIF4EBP1(S35);EIF4EBP1(T50); HNRNPK(S121); G3BP1(S230); WRNIP1(S151); CCDC86(S18); NCL(T69); NPM1(S125); HMGA1(S36); PML(S518); HMGA1(T53); NOLC1(S623); PML(S527); SRRM1(S773); SNW1(S234); SNW1(S224); PGM2L1(M171);PGM2L1(T173); SRRM1(S775); SRRM1(S597); IGF2BP1(S181); SRRM1(T614); NUCKS1(S181); ASXL3(S76); SRRM1(S777); DVL2(S480); SRRM1(S769); TPI1(S58); SRP72(S630);SRP72(T633); AKT1S1(T90); PLEC¬_HUMAN; ANXA2_HUMAN; FAS_HUMAN; LMNA_HUMAN; PDIA1_HUMAN; H4_HUMAN; 1433Z_HUMAN; PRKDC_HUMAN; AHNK_HUMAN; MYH9_HUMAN; EF2_HUMAN; P3_HUMAN; A4_HUMAN; MOES_HUMAN; H10_HUMAN; XPO2_HUMAN; SPB1_HUMAN; HSPB1_HUMAN; FLNB_HUMAN; KPYM_HUMAN; LEG1_HUMAN; H2A1A_HUMAN; CH10_HUMAN; CH60_HUMAN; HS90B_HUMAN; NPM_HUMAN; A4_HUMAN; AAAS_HUMAN; ACTBL_HUMAN; PRDX1_HUMAN; TOPK_HUMAN; H2B1B_HUMAN; 2B1B_HUMAN; G3P_HUMAN; ACTB_HUMAN; KPYM_HUMAN; HSP7C_HUMAN; K1614_HUMAN; NPL_HUMAN; ENPL_HUMAN; PDIA3_HUMAN; H2A1B_HUMAN; ACTA_HUMAN; PROF1_HUMAN; PP1A_HUMAN; ANXA1_HUMAN; EF1A1_HUMAN; TBA1C_HUMAN; NUCL_HUMAN; CH60_HUMAN; CSTA; SDC2; BEX3; SPOCK1; BEX1; RPS4Y1; VCAN; EMP1; VIM; HGD; CH13L1; TGFBI; RTL8C; CDH1; FAM20C; GTSF1; ALOXSAP; TUBB6; HLA; PPP1R27; LGALS1; CCL2; SPARC; LYZ; RAB34; HLA; TSPAN1; AS1; JUP; GYPC; TFPI; SLPI; ITM2A; GSTP1; LCN2; ALDH1A1; ABCB1; CDH1; CA2; CLEC2B; TSPAN13; IL1B; DPA1; RAB38; CAVIN2; AC093840.1; LGALS3; BEX4; PRAME and DDX60.

**8.** The method of any one of the preceding claims, wherein said expression values are obtained from phosphoproteomics, proteomics, or transcriptomics experiments.

**9.** The method of any one of the preceding claims, wherein said one or more trained predictive models are derived using machine learning or statistical learning methods, optionally wherein said one or more trained predictive models have been trained using a learning algorithm selected from random forest (rf), cubist, bayesian estimation of generalized linear models (bglm), partial least squares (pls), principal component regression (pcr), deep learning (dl) and neural network (nnet) learning algorithms.

**10.** The method of any one of the preceding claims, wherein the patient has been diagnosed with or is suspected of having cancer, optionally wherein the cancer is leukemia or a solid tumour, and optionally wherein the leukemia is acute myeloid leukemia or the solid tumour is oesophageal cancer or hepatocellular cancer.

**11.** The method of any one of the preceding claims, wherein each drug *d* is an anticancer drug.

**12.** The method of any one of the preceding claims, wherein said sample taken from said patient is a biopsy from a tumour.

**13.** A computer-implemented method of training one or more predictive models to provide a ranking of the drugs from a plurality of drugs $d_n$ in order of their predicted efficacy in a sample taken from a patient, the method comprising:

i. preparing input data comprising a plurality of drug response distance values $D_n$ for each of a plurality of drugs $d_n$, wherein *D* for each drug *d* is the difference between the distribution of expression of biological markers of sensitivity to drug *d* relative to the distribution of expression of biological markers of resistance to drug *d*;
ii. training, using the input data, one or more predictive models to provide a ranking of the drugs from said plurality of drugs $d_n$ in order of their predicted efficacy in a sample taken from a patient, optionally wherein the one or more predictive models are trained using a learning algorithm selected from random forest (rf), cubist, bayesian estimation of generalized linear models (bglm), partial least squares (pls), principal component regression (pcr), deep learning (dl) and neural network (nnet) learning algorithms;
iii. assessing, using validation data, the performance of the one or more predictive models trained according to step (ii).

**14.** A computer-readable storage medium or media storing instructions for implementing a method as claimed in any one of the preceding claims.

**15.** A system for identifying a drug with which to treat a patient, the system comprising a memory and one or more processors, wherein the one or more processors is configured to cause a method as claimed in any one of claims 1 to 12.

**Patentansprüche**

**1.** Computerimplementiertes Verfahren zum Identifizieren eines Arzneistoffs, mit dem ein Patient zu behandeln ist, wobei das Verfahren die folgenden Schritte umfasst:

a. Bereitstellen eines Datensatzes von Expressionswerten biologischer Marker aus einer von dem Patienten entnommenen Probe,
b. Verwenden des Datensatzes von Expressionswerten zum Berechnen einer Mehrzahl von Arzneistoffreaktionsdistanzwerten $D_n$ für jeden aus einer Mehrzahl von Arzneistoffen $d_n$, wobei D für jeden Arzneistoff d der Unterschied zwischen der Verteilung der Expression biologischer Marker der Sensitivität gegenüber Arzneistoff d in Bezug auf die Verteilung der Expression biologischer Marker der Resistenz gegenüber Arzneistoff d ist,
c. Bereitstellen eines oder mehrerer trainierter prädiktiver Modelle, wobei das eine oder die mehreren trainierten prädiktiven Modelle an einer Mehrzahl von Arzneistoffreaktionsdistanzwerten $D_n$ für mindestens die gleiche Mehrzahl von Arzneistoffen $d_n$ wie in Schritt b trainiert wurden,
und wobei das eine oder die mehreren trainierten prädiktiven Modelle darauf trainiert wurden, eine Rangfolge der Arzneistoffe aus der Mehrzahl von Arzneistoffen $d_n$ in der Reihenfolge ihrer vorhergesagten Wirksamkeit in der von dem Patienten entnommenen Probe bereitzustellen,
d. Eingeben der Mehrzahl von in Schritt b. erhaltenen Arzneistoffreaktionsdistanzwerten $D_n$ in das eine oder die mehreren trainierten prädiktiven Modelle und
e. Bereitstellen, unter Verwendung eines oder mehrerer der trainierten prädiktiven Modelle, einer Rangfolge der Arzneistoffe aus der Mehrzahl von Arzneistoffen $d_n$ in der Reihenfolge ihrer vorhergesagten Wirksamkeit bei dem Patienten.

**2.** Verfahren nach Anspruch 1, das ferner Folgendes umfasst:
f. Identifizieren eines Arzneistoffs, mit dem ein Patient zu behandeln ist, durch Auswählen eines der ranghöchsten Arzneistoffe, wobei der ausgewählte Arzneistoff der ranghöchste, zweitranghöchste, drittranghöchste, viertranghöchste, füntranghöchste, sechstranghöchste, siebentranghöchste, achtranghöchste, neuntranghöchste oder zehntranghöchste Arzneistoff ist.

**3.** Verfahren nach Anspruch 1 oder 2, wobei D für jeden Arzneistoff d unter Verwendung von Folgendem berechnet wird:

$$D_d = [S^{Q2}-R^{Q2}]+[S^{Q3}-R^{Q3}],$$

wobei $S^{Q2}$ und $S^{Q3}$ mediane Expressionswerte und Expressionswerte des dritten Quantils von biologischen Markern der Sensitivität gegenüber Arzneistoff d sind und $R^{Q2}$ und $R^{Q3}$ mediane Expressionswerte und Expressionswerte des dritten Quantils von biologischen Markern der Resistenz gegenüber Arzneistoff d sind.

**4.** Verfahren nach einem der Ansprüche 1 bis 3, wobei die Mehrzahl von Arzneistoffreaktionsdistanzwerten $D_n$ die mit dem D-Wert für Arzneistoff d am stärksten positiv korrelierten und am stärksten negativ korrelierten D-Werte umfasst.

**5.** Verfahren nach Anspruch 4, wobei die Mehrzahl von Arzneistoffreaktionsdistanzwerten $D_n$ eine gleiche Anzahl an mit dem D-Wert für Arzneistoff d positiv korrelierten und negativ korrelierten D-Werten umfasst, wobei optional die Mehrzahl von Arzneistoffreaktionsdistanzwerten $D_n$ die 7 am stärksten positiv und 7 am stärksten negativ mit dem D-Wert für Arzneistoff d korrelierten D-Werte umfasst.

**6.** Verfahren nach einem der vorhergehenden Ansprüche, wobei die biologischen Marker der Sensitivität gegenüber Arzneistoff d biologische Marker sind, deren Expression sich in biologischen Proben, die gegenüber Arzneistoff d sensitiv sind, konsistent als erhöht im Vergleich zu ihrer Expression in biologischen Proben, die gegenüber Arzneistoff d resistent sind, herausstellt und wobei die biologischen Marker der Resistenz gegenüber Arzneistoff d biologische Marker sind, deren Expression sich in biologischen Proben, die gegenüber Arzneistoff d resistent sind, als konsistent erhöht im Vergleich zu ihrer Expression in biologischen Proben, die gegenüber Arzneistoff d sensitiv sind, herausstellt, wobei optional die biologischen Proben:

(i) Zelllinien, optional Krebszelllinien, oder
(ii) von Patienten entnommene Primärzellen, optional von Patienten entnommene primäre Krebszellen, sind.

**7.** Verfahren nach einem der vorhergehenden Ansprüche, wobei die biologischen Marker der Sensitivität gegenüber Arzneistoff d und/oder die biologischen Marker der Resistenz gegenüber Arzneistoff d aus der aus den Folgenden bestehenden Gruppe ausgewählt sind: FAM129B(S691), SRSF6(S301), HSP90AA1(S252), SRRM2(S2102),SRRM2(T2104), DBNL(K288), DBNL(T291), EIF4EBP1(S83), EIF4EBP1(S85), EIF4EBP1(S44), LMNA(S392), TRA2A(S262), TRA2B(S266), TRA2A(S260), TRA2B(S264), LMNA(S390), ACIN1(S1004), BCLAF1(S512), MCM2(T25), HSP90AB1(S255), HSP90AB2P(S177), HSP90AB1(S255), SRRM2(M1168), SRRM2(T1177), IRF2BP2(S175), ADD1(S726), ADD2(S713), ADD3(S693), FAM129B(S691), FAM129B(S692), EIF4EBP1(Y34), EIF4EBP1(T37), EIF4EBP1(T41), EIF4EBP1(S35), EIF4EBP1(T50), HNRNPK(S121), G3BP1(S230), WRNIP1(S151), CCDC86(S18), NCL(T69), NPM1(S125), HMGA1(S36), PML(S518), HMGA1(T53), NOLC1(S623), PML(S527), SRRM1(S773), SNW1(S234), SNW1(S224), PGM2L1(M171), PGM2L1(T173), SRRM1(S775), SRRM1(S597), IGF2BP1(S181), SRRM1(T614), NUCKS1(S181), ASXL3(S76), SRRM1(S777), DVL2(S480), SRRM1(S769), TPI1(S58), SRP72(S630), SRP72(T633), AKT1S1(T90), PLEC_HUMAN, ANXA2_HUMAN, FAS_HUMAN, LMNA_HUMAN, PDIA1_HUMAN, H4_HUMAN, 1433Z_HUMAN, PRKDC_HUMAN, AHNK_HUMAN, MYH9_HUMAN, EF2_HUMAN, P3_HUMAN, A4_HUMAN, MOES_HUMAN, H10_HUMAN, XPO2_HUMAN, SPB1_HUMAN, HSPB1_HUMAN, FLNB_HUMAN, KPYM_HUMAN, LEG1_HUMAN, H2A1A _HUMAN, CH10_HUMAN, CH60_HUMAN, HS90B_HUMAN, NPM_HUMAN, A4_HUMAN, AAAS_HUMAN, ACTBL_HUMAN, PRDX1_HUMAN, TOPK_HUMAN, H2B1B_HUMAN, 2B1B_HUMAN, G3P_HUMAN, ACTB_HUMAN, KPYM_HUMAN, HSP7C_HUMAN, K1614_HUMAN, NPL_HUMAN, ENPL_HUMAN, PDIA3_HUMAN, H2A1B_HUMAN, ACTA_HUMAN, PROF1_HUMAN, PP1A_HUMAN, ANXA1_HUMAN, EF1A1_HUMAN, TBA1C_HUMAN, NUCL_HUMAN, CH60_HUMAN, CSTA, SDC2, BEX3, SPOCK1, BEX1, RPS4Y1, VCAN, EMP1, VIM, HGD, CH13L1, TGFBI, RTL8C, CDH1, FAM20C, GTSF1, ALOX5AP, TUBB6, HLA, PPP1R27, LGALS1, CCL2,

SPARC, LYZ, RAB34, HLA, TSPAN1, AS1, JUP, GYPC, TFPI, SLPI, ITM2A, GSTP1, LCN2, ALDH1A1, ABCB1, CDH1, CA2, CLEC2B, TSPAN13, IL1B, DPA1, RAB38, CAVIN2, AC093840.1, LGALS3, BEX4, PRAME und DDX60.

**8.** Verfahren nach einem der vorhergehenden Ansprüche, wobei die Expressionswerte aus Phosphoproteomik-, Proteomik- oder Transkriptomik-Experimenten erhalten werden.

**9.** Verfahren nach einem der vorhergehenden Ansprüche, wobei das eine oder die mehreren trainierten prädiktiven Modelle unter Verwendung von Maschinenlernen oder statistischen Lernverfahren erhalten werden, wobei optional das eine oder die mehreren trainierten prädiktiven Modelle unter Verwendung eines Lernalgorithmus trainiert wurden, der aus den Lernalgorithmen Random Forest (rf), Cubist, Bayes'sche Schätzung generalisierter linearer Modelle (bglm), partielle kleinste Quadrate (pls), Hauptkomponentenregression (pcr), Deep Learning (dl) und neuronales Netzwerk (nnet) ausgewählt ist.

**10.** Verfahren nach einem der vorhergehenden Ansprüche, wobei bei dem Patienten Krebs diagnostiziert wurde oder vermutet wird, wobei optional der Krebs Leukämie oder ein solider Tumor ist und wobei optional die Leukämie akute myeloische Leukämie ist oder der solide Tumor Speiseröhrenkrebs oder hepatozellulärer Krebs ist.

**11.** Verfahren nach einem der vorhergehenden Ansprüche, wobei jeder Arzneistoff d ein Antikrebs-Arzneistoff ist.

**12.** Verfahren nach einem der vorhergehenden Ansprüche, wobei die von dem Patienten entnommene Probe eine Biopsie aus einem Tumor ist.

**13.** Computerimplementiertes Verfahren zum Trainieren eines oder mehrerer prädiktiver Modelle, um eine Rangfolge der Arzneistoffe aus einer Mehrzahl von Arzneistoffen $d_n$ in der Reihenfolge ihrer vorhergesagten Wirksamkeit in einer von einem Patienten entnommenen Probe bereitzustellen, wobei das Verfahren Folgendes umfasst:

i. Vorbereiten von Eingabedaten, die eine Mehrzahl von Arzneistoffreaktionsdistanzwerten $D_n$ für jeden aus einer Mehrzahl von Arzneistoffen $d_n$ umfassen, wobei D für jeden Arzneistoff d der Unterschied zwischen der Verteilung der Expression biologischer Marker der Sensitivität gegenüber Arzneistoff d in Bezug auf die Verteilung der Expression biologischer Marker der Resistenz gegenüber Arzneistoff d ist,
ii. Trainieren, unter Verwendung der Eingabedaten, eines oder mehrerer prädiktiver Modelle, um eine Rangfolge der Arzneistoffe aus der Mehrzahl von Arzneistoffen $d_n$ in der Reihenfolge ihrer vorhergesagten Wirksamkeit in einer von einem Patienten entnommenen Probe bereitzustellen, wobei optional das eine oder die mehreren prädiktiven Modelle unter Verwendung eines Lernalgorithmus trainiert werden, der aus den Lernalgorithmen Random Forest (rf), Cubist, Bayes'sche Schätzung generalisierter linearer Modelle (bglm), partielle kleinste Quadrate (pls), Hauptkomponentenregression (pcr), Deep Learning (dl) und neuronales Netzwerk (nnet) ausgewählt ist,
iii. Beurteilen, unter Verwendung von Validierungsdaten, der Leistung des einen oder der mehreren gemäß Schritt (ii) trainierten prädiktiven Modelle.

**14.** Computerlesbares Speichermedium oder computerlesbare Speichermedien, das bzw. die Anweisungen zum Implementieren eines Verfahrens nach einem der vorhergehenden Ansprüche speichert bzw. speichern.

**15.** System zum Identifizieren eines Arzneistoffs, mit dem ein Patient zu behandeln ist, wobei das System einen Speicher und einen oder mehrere Prozessoren umfasst, wobei der eine oder die mehreren Prozessoren dafür ausgelegt sind, ein Verfahren nach einem der Ansprüche 1 bis 12 zu veranlassen.

## Revendications

**1.** Procédé mis en œuvre par ordinateur d'identification d'un médicament avec lequel traiter un patient, le procédé comprenant les étapes de :

a. fourniture d'un ensemble de données de valeurs d'expression de marqueurs biologiques à partir d'un échantillon prélevé sur ledit patient ;
b. utilisation dudit ensemble de données de valeurs d'expression pour calculer une pluralité de valeurs de distance de réponse au médicament $D_n$ pour chacun d'une pluralité de médicaments $d_n$, D pour chaque médicament d étant la différence entre la distribution d'expressions de marqueurs biologiques de sensibilité à un médicament d par rapport à la distribution d'expressions de marqueurs biologiques de résistance à un médicament d ;

c. fourniture d'un ou plusieurs modèles prédictifs entraînés, le ou les modèles prédictifs entraînés ayant été entraînés sur une pluralité de valeurs de distance de réponse au médicament $D_n$ pour au moins la même pluralité de médicaments $d_n$ que dans l'étape b ;

et le ou les modèles prédictifs entraînés ayant été entraînés pour fournir un classement des médicaments à partir de ladite pluralité de médicaments $d_n$ par ordre de leur efficacité prédite dans ledit échantillon prélevé sur ledit patient ;

d. saisie de ladite pluralité de valeurs de distance de réponse au médicament $D_n$ obtenue à l'étape b. dans lesdits un ou plusieurs modèles prédictifs entraînés ; et

e. fourniture, à l'aide d'un ou plusieurs des modèles prédictifs entraînés, d'un classement des médicaments de ladite pluralité de médicaments $d_n$ par ordre de leur efficacité prédite chez ledit patient.

**2.** Procédé selon la revendication 1, comprenant en outre :
f. identification d'un médicament avec lequel traiter un patient en sélectionnant un des médicaments de rang le plus élevé, le médicament sélectionné étant le plus élevé, deuxième le plus élevé, troisième le plus élevé, quatrième le plus élevé, cinquième le plus élevé, sixième le plus élevé, septième le plus élevé, huitième le plus élevé, neuvième le plus élevé ou dixième le plus élevé.

**3.** Procédé selon la revendication 1 ou 2, dans lequel D est calculé pour chaque médicament d en utilisant :

$$D_d = [S^{Q2}-R^{Q2}]+[S^{Q3}-R^{Q3}],$$

où $S^{Q2}$ et $S^{Q3}$ sont des valeurs d'expression médiane et de troisième quantile de marqueurs biologiques de sensibilité au médicament d ; et $R^{Q2}$ et $R^{Q3}$ sont des valeurs d'expression médiane et de troisième quantile de marqueurs biologiques de résistance au médicament d.

**4.** Procédé selon l'une quelconque des revendications 1 à 3, dans lequel ladite pluralité de valeurs de distance de réponse au médicament $D_n$ comprend les valeurs D les plus corrélées positivement et les plus corrélées négativement à la valeur D pour le médicament d.

**5.** Procédé selon la revendication 4, dans lequel ladite pluralité de valeurs de distance de réponse au médicament $D_n$ comprend un nombre égal de valeurs D corrélées positivement et corrélées négativement à la valeur D pour le médicament d, éventuellement dans lequel ladite pluralité de valeurs de distance de réponse au médicament $D_n$ comprend les 7 valeurs D les plus corrélées positivement et les 7 valeurs D les plus corrélées négativement à la valeur D pour le médicament d.

**6.** Procédé selon l'une quelconque des revendications précédentes, dans lequel lesdits marqueurs biologiques de sensibilité au médicament d sont des marqueurs biologiques dont l'expression est constamment observée comme étant augmentée dans des échantillons biologiques sensibles au médicament d par rapport à leur expression dans des échantillons biologiques résistants au médicament d, et dans lequel lesdits marqueurs biologiques de résistance au médicament d sont des marqueurs biologiques dont l'expression est constamment observée comme étant augmentée dans des échantillons biologiques résistants au médicament d par rapport à leur expression dans des échantillons biologiques sensibles au médicament d, éventuellement dans lequel les échantillons biologiques sont :

(i) des lignées cellulaires, éventuellement des lignées cellulaires de cancer ; ou
(ii) des cellules primaires obtenues à partir de patients, éventuellement des cellules cancéreuses primaires obtenues à partir de patients.

**7.** Procédé selon l'une quelconque des revendications précédentes, dans lequel lesdits marqueurs biologiques de sensibilité au médicament d et/ou lesdits marqueurs biologiques de résistance au médicament d sont choisis dans le groupe constitué de : FAM129B(S691) ; SRSF6(S301) ; HSP90AA1(S252) ; SRRM2(S2102) ; SRRM2(T2104) ; DBNL(K288) ; DBNL(T291) ; EIF4EBP1(S83) ; EIF4EBP1(S85) ; EIF4EBP1(S44) ; LMNA(S392) ; TRA2A(S262) ; TRA2B(S266) ; TRA2A(S260) ; TRA2B(S264) ; LMNA(S390) ; ACIN1(S1004) ; BCLAF1(S512) ; MCM2(T25) ; HSP90AB1(S255) ; HSP90AB2P(S177) ; HSP90AB1(S255) ; SRRM2(M1168) ; SRRM2(T1177) ; IRF2BP2(S175) ; ADD1(S726) ; ADD2(S713) ; ADD3(S693) ; FAM129B(S691) ; FAM129B(S692) ; EIF4EBP1(Y34) ; EIF4EBP1(T37) ; EIF4EBP1(T41) ; EIF4EBP1(S35) ; EIF4EBP1(T50) ; HNRNPK(S121) ; G3BP1(S230) ; WRNIP1(S151) ;

CCDC86(S18) ; NCL(T69) ; NPM1(S125) ; HMGA1(S36) ; PML(S518) ; HMGA1(T53) ; NOLC1(S623) ; PML(S527) ; SRRM1(S773) ; SNW1(S234) ; SNW1(S224) ; PGM2L1(M171) ; PGM2L1(T173) ; SRRM1(S775) ; SRRM1(S597) ; IGF2BP1(S181) ; SRRM1(T614) ; NUCKS1(S181) ; ASXL3(S76) ; SRRM1(S777) ; DVL2(S480) ; SRRM1(S769) ; TPI1(S58) ; SRP72(S630) ; SRP72(T633) ; AKT1S1(T90) ; PLEC_HUMAN ; ANXA2_HUMAN ; FAS_HUMAN ; LMNA_HUMAN ; PDIA1_HUMAN ; H4_HUMAN ; 1433Z_HUMAN ; PRKDC_HUMAN ; AHNK_HUMAN ; MYH9_HUMAN ; EF2_HUMAN ; P3_HUMAN ; A4_HUMAN ; MOES_HUMAN ; H10_HUMAN ; XPO2_HUMAN ; SPB1_HUMAN ; HSPB1_HUMAN ; FLNB_HUMAN ; KPYM_HUMAN ; LEG1_HUMAN ; H2A1A _HUMAN ; CH10_HUMAN ; CH60_HUMAN ; HS90B_HUMAN ; NPM _HUMAN ; A4_HUMAN ; AAAS_HUMAN ; ACTBL_HUMAN ; PRDX1_HUMAN ; TOPK_HUMAN ; H2B1B_HUMAN ; 2B1B_HUMAN ; G3P_HUMAN ; ACTB_HUMAN ; KPYM_HUMAN ; HSP7C_HUMAN ; K1614_HUMAN ; NPL_HUMAN ; ENPL_HUMAN ; PDIA3_HUMAN ; H2A1B_HUMAN ; ACTA_HUMAN ; PROF1_HUMAN ; PP1A_HUMAN ; ANXA1_HUMAN ; EF1A1_HUMAN ; TBA1C_HUMAN ; NUCL_HUMAN ; CH60_HUMAN ; CSTA ; SDC2 ; BEX3 ; SPOCK1 ; BEX1 ; RPS4Y1 ; VCAN ; EMP1 ; VIM ; HGD ; CH13L1 ; TGFBI ; RTL8C ; CDH1 ; FAM20C ; GTSF1 ; ALOX5AP ; TUBB6 ; HLA ; PPP1R27 ; LGALS1 ; CCL2 ; SPARC ; LYZ ; RAB34 ; HLA ; TSPAN1 ; AS1 ; JUP ; GYPC ; TFPI ; SLPI ; ITM2A ; GSTP1 ; LCN2 ; ALDH1A1 ; ABCB1 ; CDH1 ; CA2 ; CLEC2B ; TSPAN13 ; IL1B ; DPA1 ; RAB38 ; CAVIN2 ; AC093840.1 ; LGALS3 ; BEX4 ; PRAME et DDX60.

**8.** Procédé selon l'une quelconque des revendications précédentes, dans lequel lesdites valeurs d'expression sont obtenues à partir d'expériences phosphoprotéomiques, protéomiques ou transcriptomiques.

**9.** Procédé selon l'une quelconque des revendications précédentes, dans lequel lesdits un ou plusieurs modèles prédictifs entraînés sont dérivés en utilisant des procédés d'apprentissage automatique ou d'apprentissage statistique, éventuellement dans lequel lesdits un ou plusieurs modèles prédictifs entraînés ont été entraînés en utilisant un algorithme d'apprentissage sélectionné parmi des algorithmes d'apprentissage de forêt aléatoire (rf), cubiste, estimation bayésienne des modèles linéaires généralisés (bglm), moindres carrés partiels (pls), régression par composantes principales (pcr), apprentissage profond (dl) et réseau neuronal (nnet).

**10.** Procédé selon l'une quelconque des revendications précédentes, dans lequel le patient a été diagnostiqué ou est suspecté d'avoir un cancer, éventuellement dans lequel le cancer est une leucémie ou une tumeur solide, et éventuellement dans lequel la leucémie est une leucémie myéloïde aiguë ou la tumeur solide est un cancer de l'œsophage ou un cancer hépatocellulaire.

**11.** Procédé selon l'une quelconque des revendications précédentes, dans lequel chaque médicament d est un médicament anticancéreux.

**12.** Procédé selon l'une quelconque des revendications précédentes, dans lequel ledit échantillon prélevé sur ledit patient est une biopsie d'une tumeur.

**13.** Procédé mis en œuvre par ordinateur d'entraînement d'un ou plusieurs modèles prédictifs pour fournir un classement des médicaments à partir d'une pluralité de médicaments $d_n$ par ordre de leur efficacité prédite dans un échantillon prélevé sur un patient, le procédé comprenant :

i. préparation de données d'entrée comprenant une pluralité de valeurs de distance de réponse au médicament $D_n$ pour chacun d'une pluralité de médicaments $d_n$, dans laquelle D pour chaque médicament d est la différence entre la distribution d'expressions de marqueurs biologiques de sensibilité au médicament d par rapport à la distribution d'expressions de marqueurs biologiques de résistance au médicament d ;
ii. entraînement, à l'aide des données d'entrée, d'un ou plusieurs modèles prédictifs pour fournir un classement des médicaments de ladite pluralité de médicaments $d_n$ par ordre de leur efficacité prédite dans un échantillon prélevé sur un patient, éventuellement dans lequel le ou les modèles prédictifs sont entraînés à l'aide d'un algorithme d'apprentissage sélectionné parmi des algorithmes d'apprentissage de forêt aléatoire (rf), cubiste, estimation bayésienne des modèles linéaires généralisés (bglm), moindres carrés partiels (pls), régression par composantes principales (pcr), apprentissage profond (dl) et réseau neuronal (nnet) ;
iii. évaluation, à l'aide de données de validation, de la performance du ou des modèles prédictifs entraînés selon l'étape (ii).

**14.** Support ou supports d'enregistrement lisible(s) par ordinateur mémorisant des instructions pour la mise en œuvre d'un procédé selon l'une quelconque des revendications précédentes.

15. Système pour identifier un médicament avec lequel traiter un patient, le système comprenant une mémoire et un ou plusieurs processeurs, dans lequel le ou les processeurs sont configurés pour provoquer un procédé selon l'une quelconque des revendications 1 à 12.

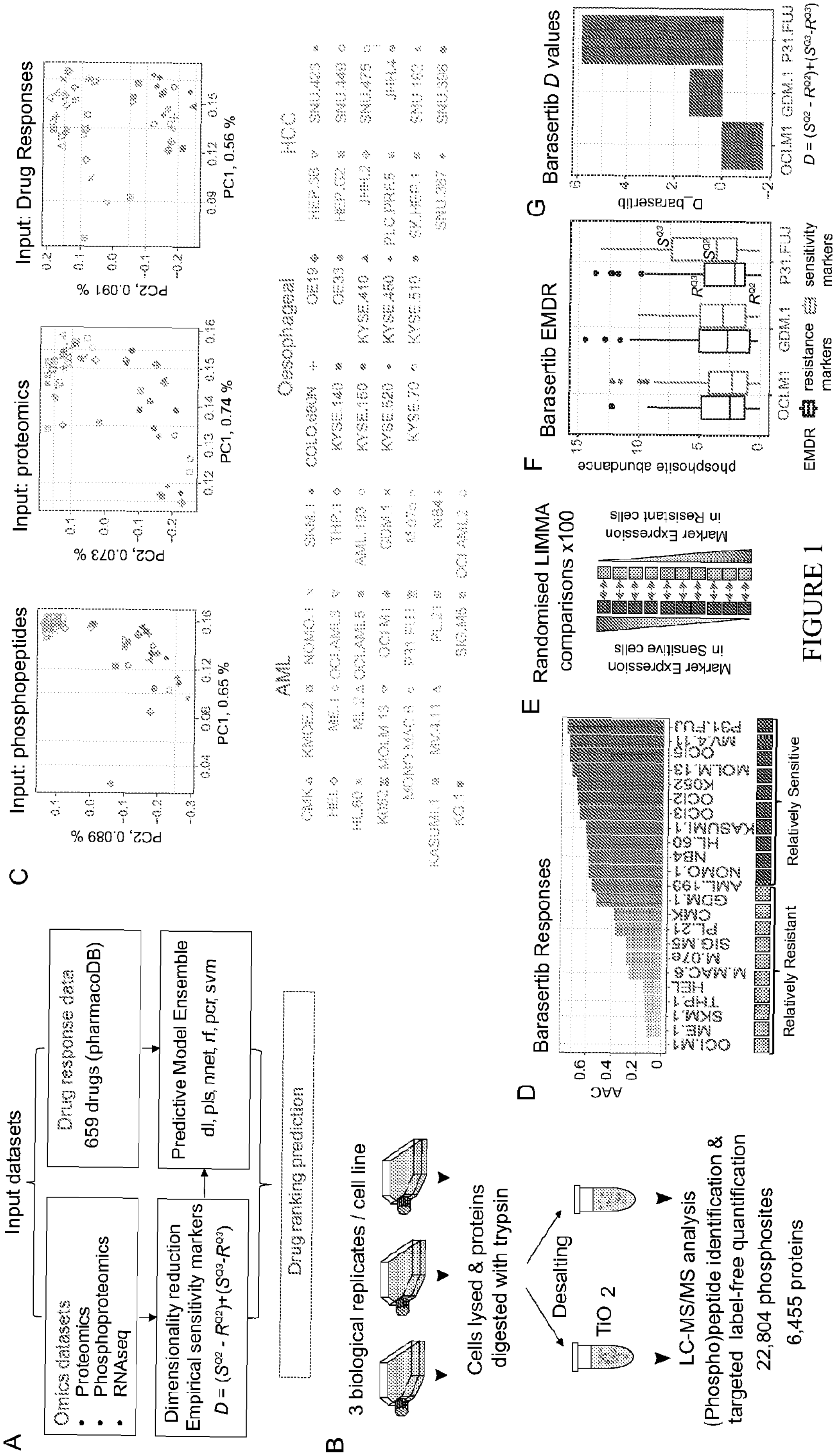
A
Input datasets
Omics datasets
Proteomics
Phosphoproteomics
RNAseq
Drug response data
659 drugs (pharmacoDB)
Dimensionality reduction
Empirical sensitivity markers
D = (S^Q2 - R^Q2)+(S^Q3-R^Q3)
Predictive Model Ensemble
dl, pls, nnet, rf, pcr, svm
Drug ranking prediction
B
3 biological replicates / cell line
Cells lysed & proteins digested with trypsin
TiO 2
Desalting
LC-MS/MS analysis
(Phospho)peptide identification & targeted label-free quantification
22,804 phosphosites
6,455 proteins
C
Input: phosphopeptides
Input: proteomics
Input: Drug Responses
PC1, 0.65 %
PC2, 0.089 %
PC1, 0.74 %
PC2, 0.073 %
PC1, 0.56 %
PC2, 0.091 %
AML
Oesophageal
HCC
D
Barasertib Responses
AAC
Relatively Resistant
Relatively Sensitive
E
Randomised LIMMA comparisons x100
Marker Expression in Sensitive cells
Marker Expression in Resistant cells
F
Barasertib EMDR
phosphosite abundance
EMDR
resistance markers
sensitivity markers
G
Barasertib D values
D_barasertib
D = (S^Q2 - R^Q2)+(S^Q3-R^Q3)

FIGURE 1

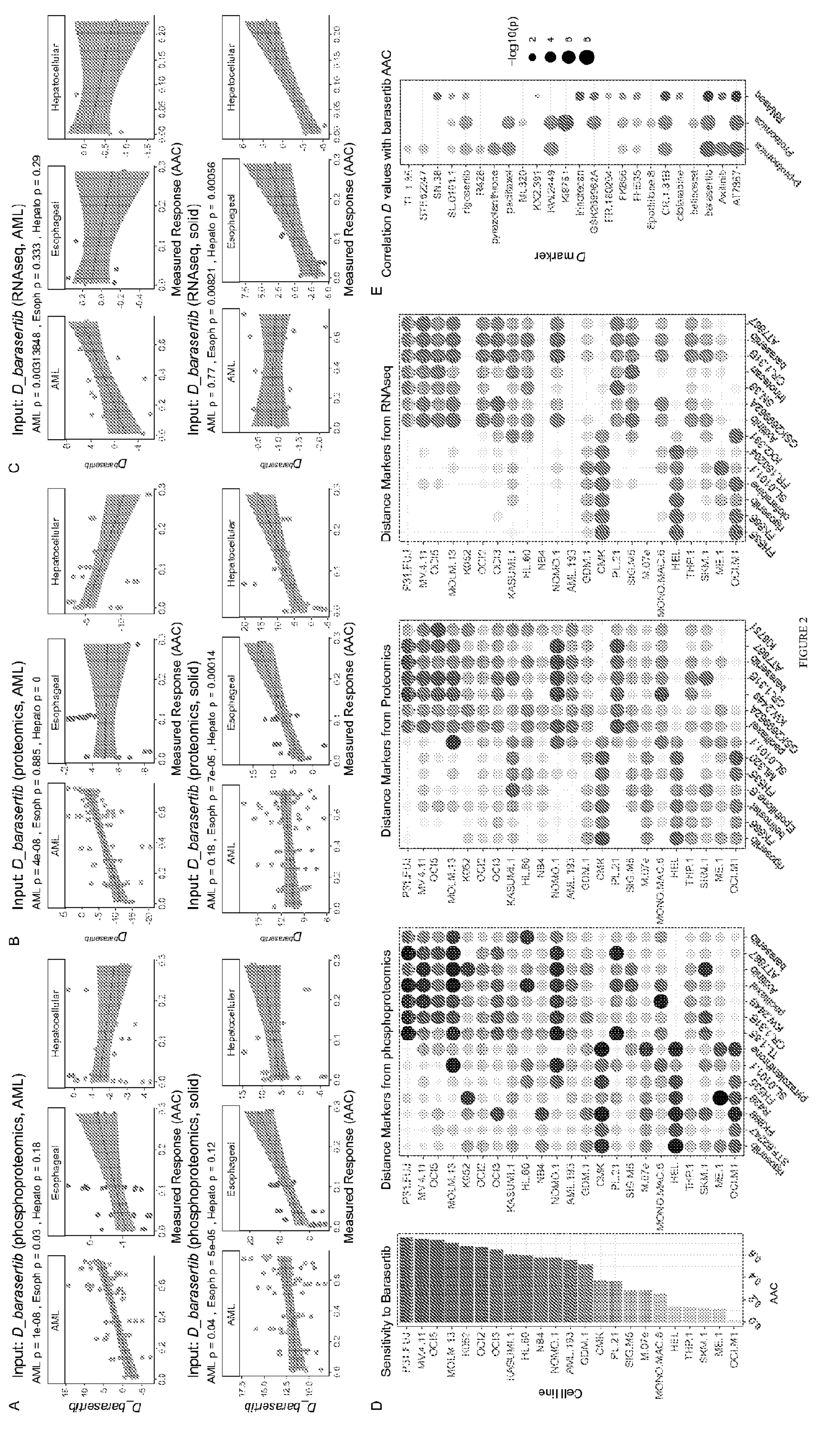
A
Input: D_barasertib (phosphoproteomics, AML)
AML p = 1e-08 , Esoph p = 0.03 , Hepato p = 0.18
Input: D_barasertib (phosphoproteomics, solid)
AML p = 0.04 , Esoph p = 5e-05 , Hepato p = 0.12
B
Input: D_barasertib (proteomics, AML)
AML p = 4e-08 , Esoph p = 0.885 , Hepato p = 0
Input: D_barasertib (proteomics, solid)
AML p = 0.18 , Esoph p = 7e-05 , Hepato p = 0.00014
C
Input: D_barasertib (RNAseq, AML)
AML p = 0.0313848 , Esoph p = 0.333 , Hepato p = 0.29
Input: D_barasertib (RNAseq, solid)
AML p = 0.77 , Esoph p = 0.00821 , Hepato p = 0.00056
AML
Esophageal
Hepatocellular
Measured Response (AAC)
D
Sensitivity to Barasertib
Cell line
AAC
Distance Markers from phosphoproteomics
Distance Markers from Proteomics
Distance Markers from RNAseq
E
Correlation D values with barasertib AAC
D marker
-log10(p)

FIGURE 2

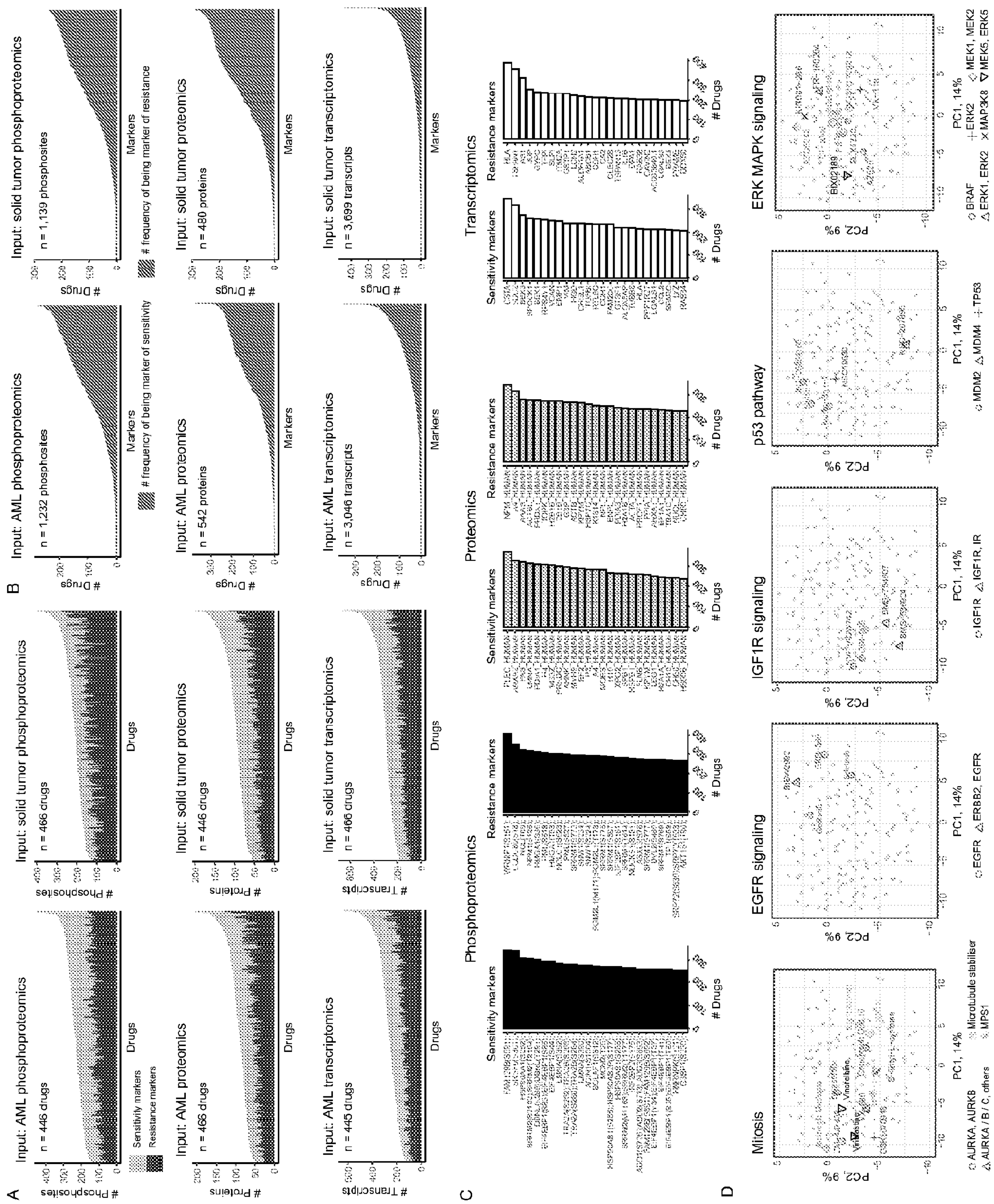
A
Input: AML phosphoproteomics
n = 446 drugs
Input: solid tumor phosphoproteomics
n = 466 drugs
Input: AML proteomics
n = 466 drugs
Input: solid tumor proteomics
n = 446 drugs
Input: AML transcriptomics
n = 445 drugs
Input: solid tumor transcriptomics
n = 466 drugs
# Phosphosites
# Proteins
# Transcripts
Drugs
Sensitivity markers
Resistance markers
B
Input: AML phosphoproteomics
n = 1,232 phosphosites
Input: solid tumor phosphoproteomics
n = 1,139 phosphosites
Input: AML proteomics
n = 542 proteins
Input: solid tumor proteomics
n = 480 proteins
Input: AML transcriptomics
n = 3,046 transcripts
Input: solid tumor transcriptomics
n = 3,699 transcripts
# Drugs
Markers
# frequency of being marker of sensitivity
# frequency of being marker of resistance
C
Phosphoproteomics
Proteomics
Transcriptomics
Sensitivity markers
Resistance markers
# Drugs
D
Mitosis
EGFR signaling
IGF1R signaling
p53 pathway
ERK MAPK signaling
PC1, 14%
PC2, 9%
Vinorelbine
AURKA, AURKB
AURKA / B / C, others
AURKA, AURKC
KIF11
Microtubule stabiliser
MPS1
KSP
Microtubule destabiliser
EGFR
ERBB2, EGFR
IGF1R
IGF1R, IR
MDM2
MDM4
TP53
BRAF
ERK1, ERK2
ERK2
MAP3K8
MEK1, MEK2
MEK5, ERK5
BIX02189

FIGURE 3

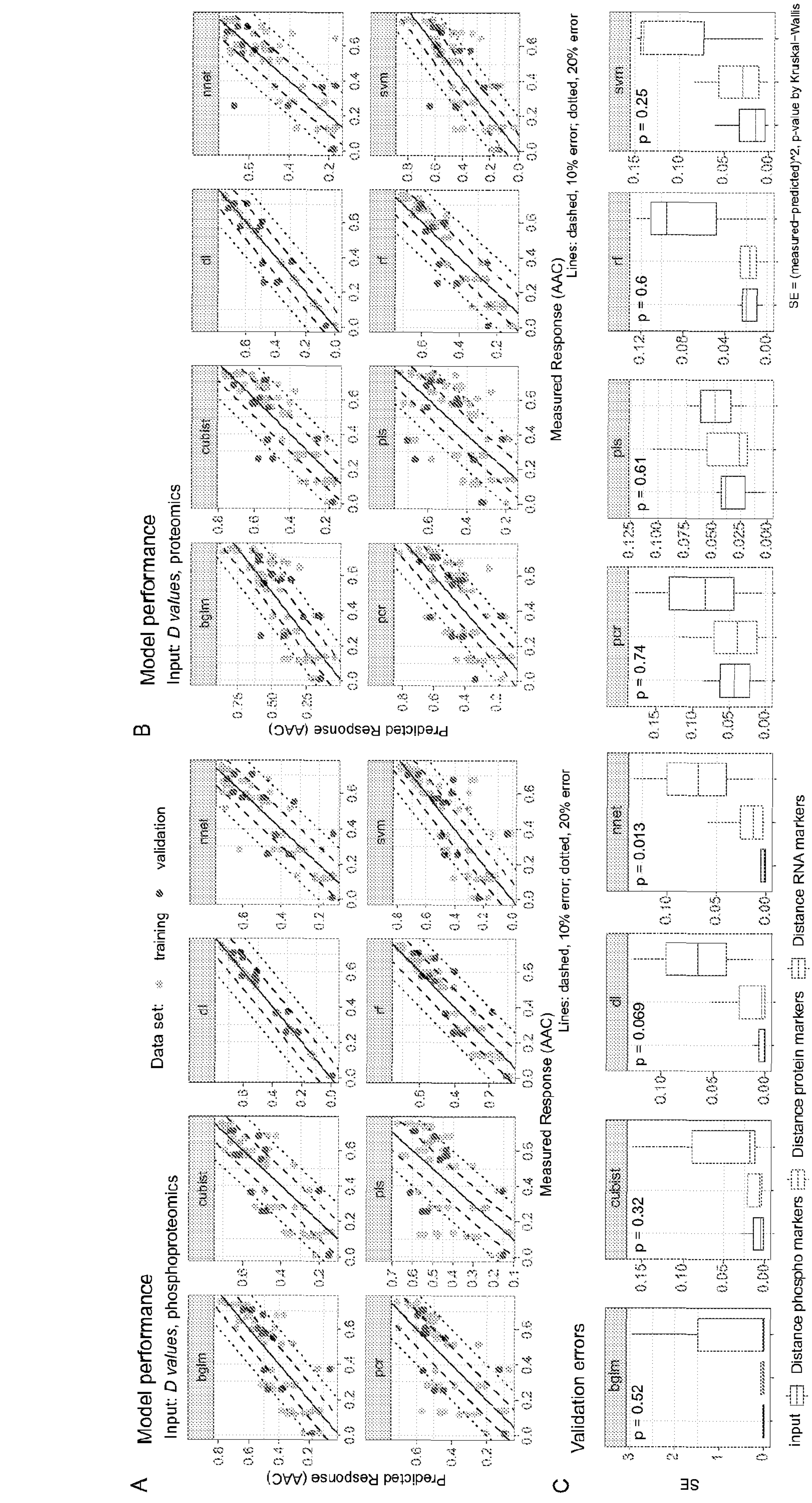
A
Model performance
Input: D values, phosphoproteomics
Data set: training validation
bglm
cubist
dl
nnet
pcr
pls
rf
svm
Predicted Response (AAC)
Measured Response (AAC)
Lines: dashed, 10% error; dotted, 20% error
B
Model performance
Input: D values, proteomics
C
Validation errors
SE
p = 0.52
p = 0.32
p = 0.069
p = 0.013
p = 0.74
p = 0.61
p = 0.6
p = 0.25
input Distance phospho markers Distance protein markers Distance RNA markers
SE = (measured−predicted)^2, p-value by Kruskal−Wallis

FIGURE 4

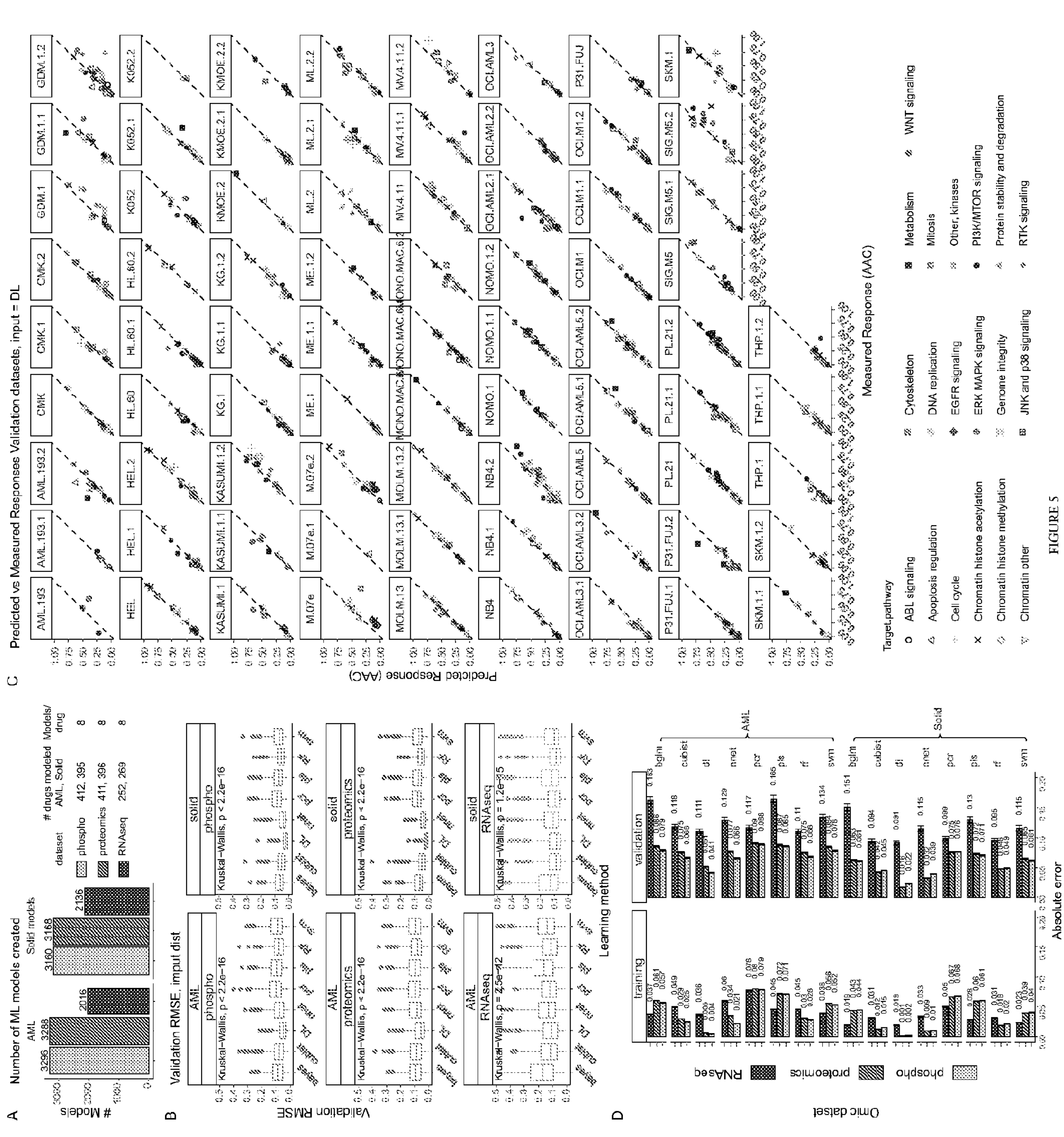
A
Number of ML models created
B
Validation RMSE, imput dist
Learning method
C
Predicted vs Measured Responses Validation datasets, input = DL
Predicted Response (AAC)
Measured Response (AAC)
D
Omic dataset
Absolute error
Target.pathway

FIGURE 5

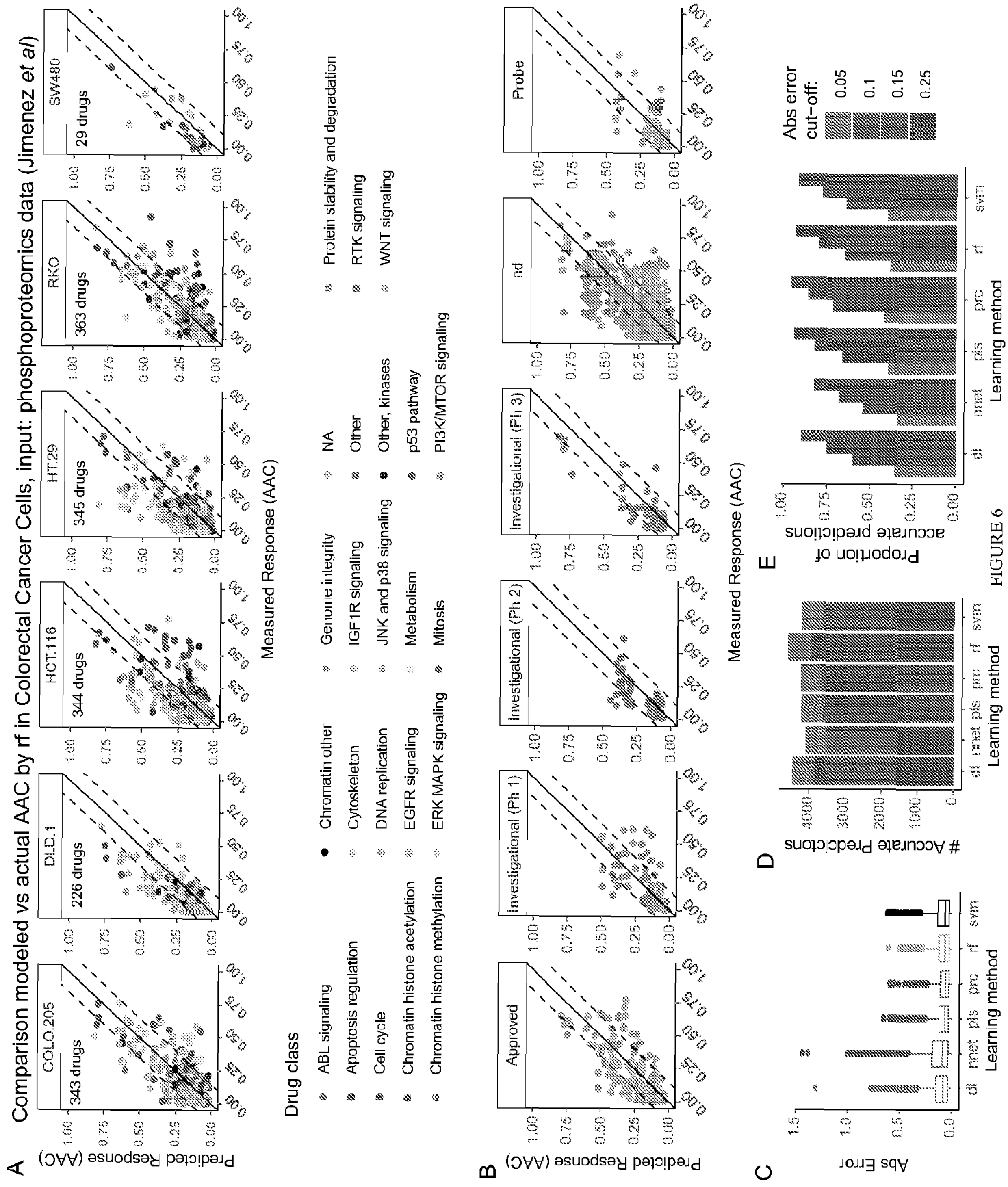
A
Comparison modeled vs actual AAC by rf in Colorectal Cancer Cells, input: phosphoproteomics data (Jimenez et al)
COLO.205
343 drugs
DLD.1
226 drugs
HCT.116
344 drugs
HT.29
345 drugs
RKO
363 drugs
SW480
29 drugs
Predicted Response (AAC)
Measured Response (AAC)
Drug class
ABL signaling
Apoptosis regulation
Cell cycle
Chromatin histone acetylation
Chromatin histone methylation
Chromatin other
Cytoskeleton
DNA replication
EGFR signaling
ERK MAPK signaling
Genome integrity
IGF1R signaling
JNK and p38 signaling
Metabolism
Mitosis
NA
Other
Other, kinases
p53 pathway
PI3K/MTOR signaling
Protein stability and degradation
RTK signaling
WNT signaling
B
Approved
Investigational (Ph 1)
Investigational (Ph 2)
Investigational (Ph 3)
nd
Probe
C
Abs Error
Learning method
D
# Accurate Predictions
E
Proportion of accurate predictions
Abs error cut-off:
0.05
0.1
0.15
0.25

FIGURE 6

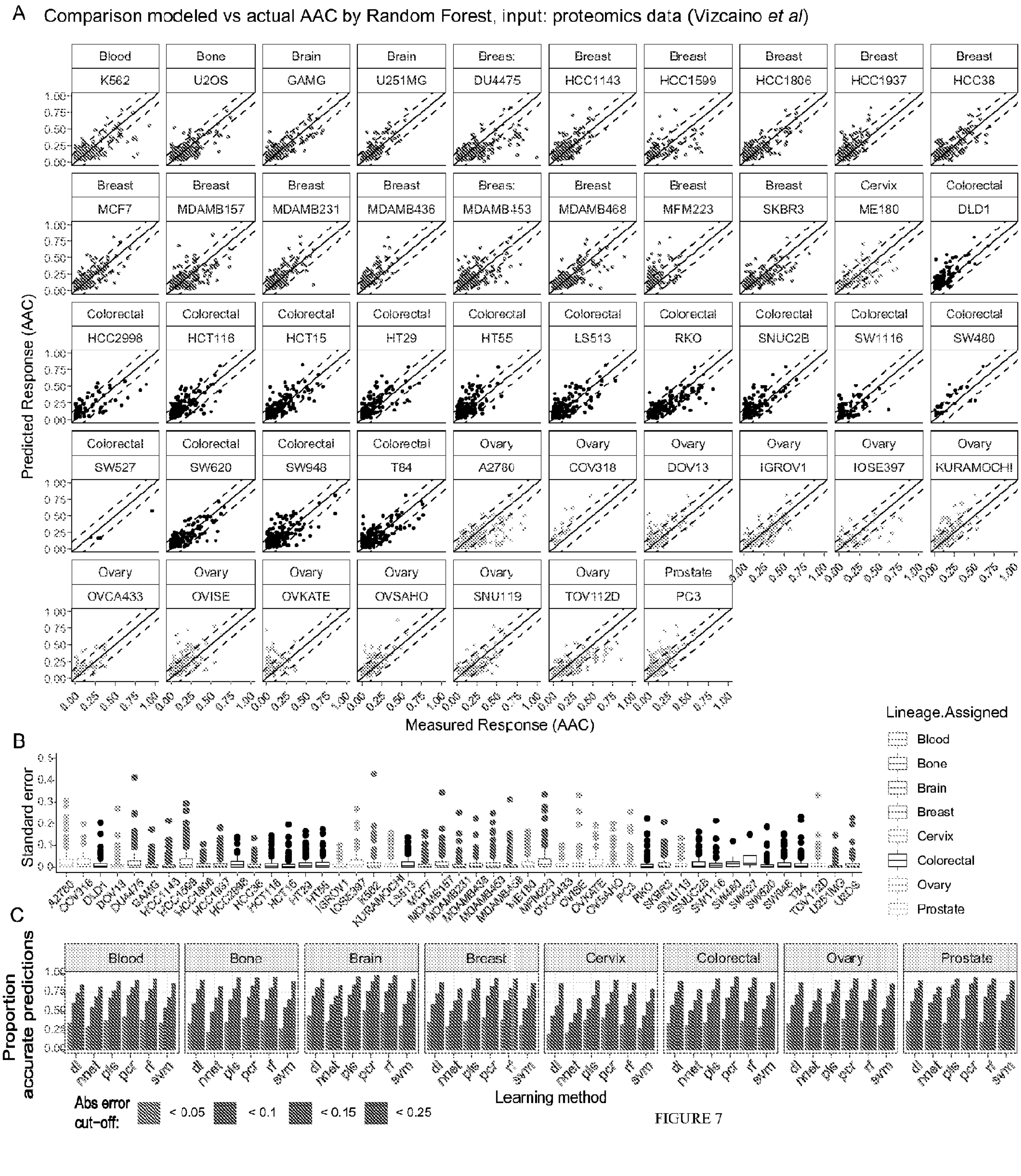
A Comparison modeled vs actual AAC by Random Forest, input: proteomics data (Vizcaino et al)
Predicted Response (AAC)
Measured Response (AAC)
Lineage.Assigned
Blood
Bone
Brain
Breast
Cervix
Colorectal
Ovary
Prostate
B
Standard error
C
Proportion accurate predictions
Learning method
Abs error cut-off:
< 0.05
< 0.1
< 0.15
< 0.25

FIGURE 7

A

Qualitative data in TP cell lines

| parameter | value |
|---|---|
| Number of peptides | 93251 |
| Unique peptides | 80770 |
| Unique Proteins | 10419 |
| Mascot scr (no mod)* | 61 +/− 28 |

Qualitative data in PP cell lines

| parameter | value |
|---|---|
| Number of peptides | 41043 |
| Unique peptides | 34585 |
| Total phosphopeptides | 31275 |
| Unique phosphopeptides | 25638 |
| Unique S/T peptides | 23930 |
| Unique Y peptides | 2130 |
| Unique Proteins | 6919 |
| Unique Phosphoproteins | 5744 |
| Mascot scr (no mod)* | 54 +/− 25 |
| Mascot scr (phospho)* | 54 +/− 25 |

** Average +/− SD*

B

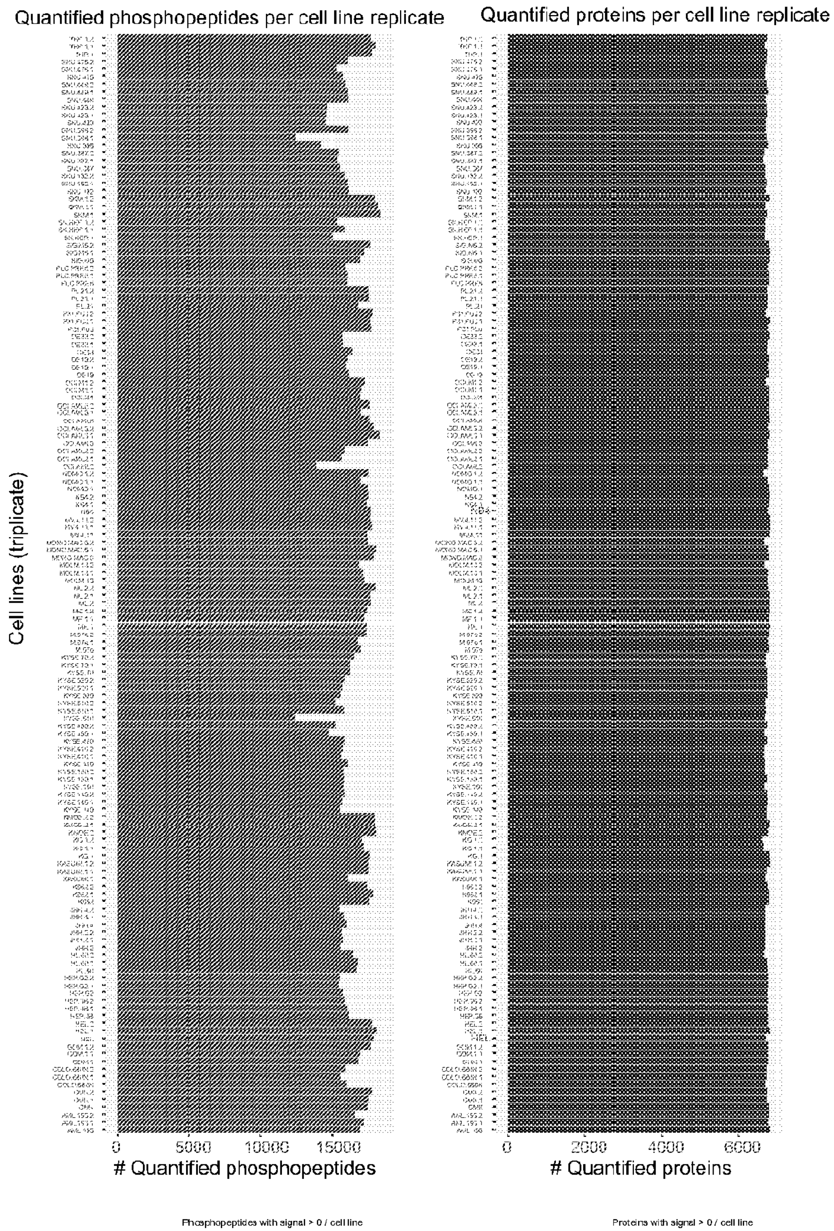


FIGURE 8

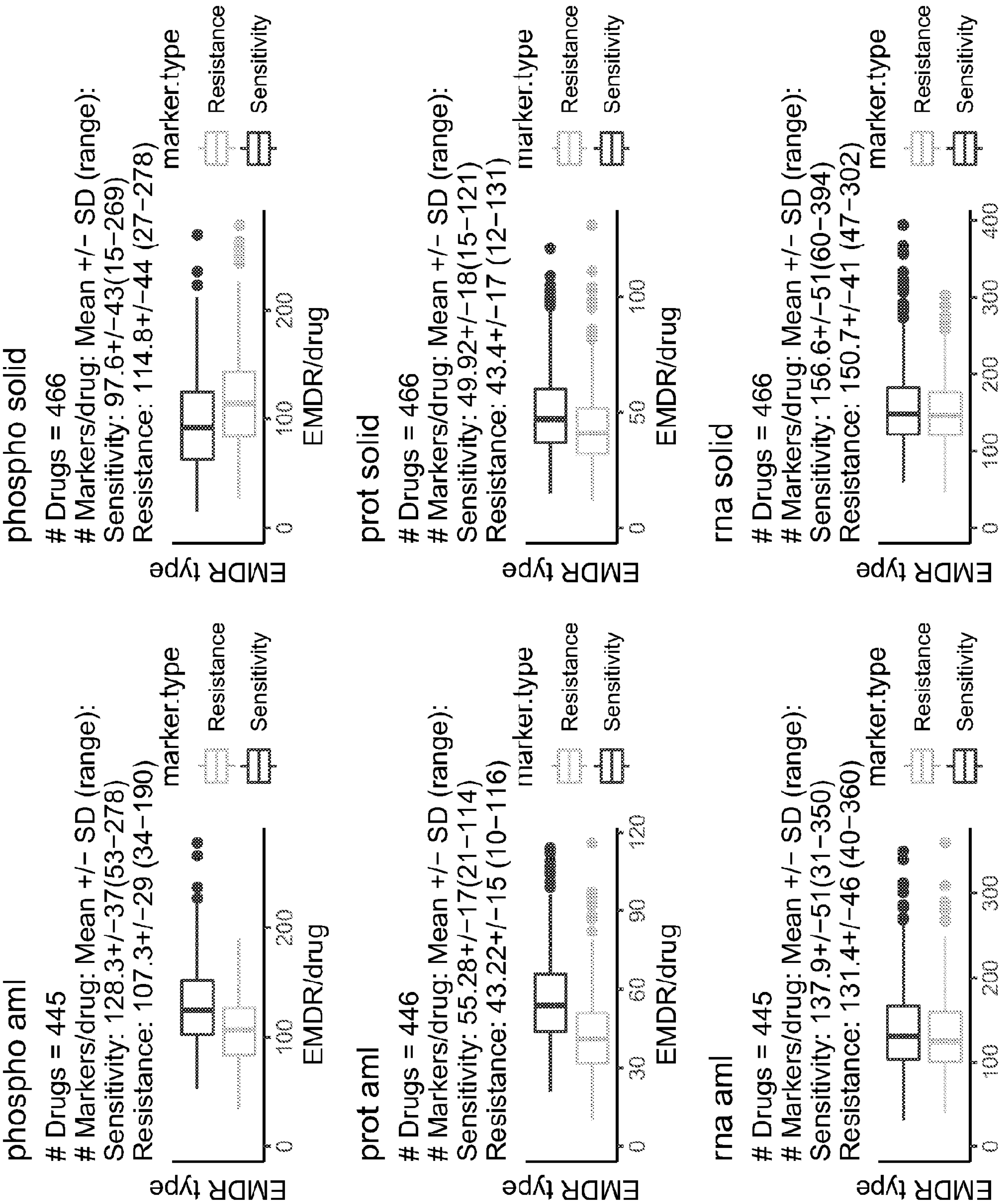
phospho aml
# Drugs = 445
# Markers/drug: Mean +/− SD (range):
Sensitivity: 128.3+/−37(53−278)
Resistance: 107.3+/−29 (34−190)
EMDR type
EMDR/drug
0
100
200
marker.type
Resistance
Sensitivity
phospho solid
# Drugs = 466
# Markers/drug: Mean +/− SD (range):
Sensitivity: 97.6+/−43(15−269)
Resistance: 114.8+/−44 (27−278)
EMDR type
EMDR/drug
0
100
200
marker.type
Resistance
Sensitivity
prot aml
# Drugs = 446
# Markers/drug: Mean +/− SD (range):
Sensitivity: 55.28+/−17(21−114)
Resistance: 43.22+/−15 (10−116)
EMDR type
EMDR/drug
0
30
60
90
120
marker.type
Resistance
Sensitivity
prot solid
# Drugs = 466
# Markers/drug: Mean +/− SD (range):
Sensitivity: 49.92+/−18(15−121)
Resistance: 43.4+/−17 (12−131)
EMDR type
EMDR/drug
0
50
100
marker.type
Resistance
Sensitivity
rna aml
# Drugs = 445
# Markers/drug: Mean +/− SD (range):
Sensitivity: 137.9+/−51(31−350)
Resistance: 131.4+/−46 (40−360)
EMDR type
EMDR/drug
0
100
200
300
marker.type
Resistance
Sensitivity
rna solid
# Drugs = 466
# Markers/drug: Mean +/− SD (range):
Sensitivity: 156.6+/−51(60−394)
Resistance: 150.7+/−41 (47−302)
EMDR type
EMDR/drug
0
100
200
300
400
marker.type
Resistance
Sensitivity

FIGURE 9

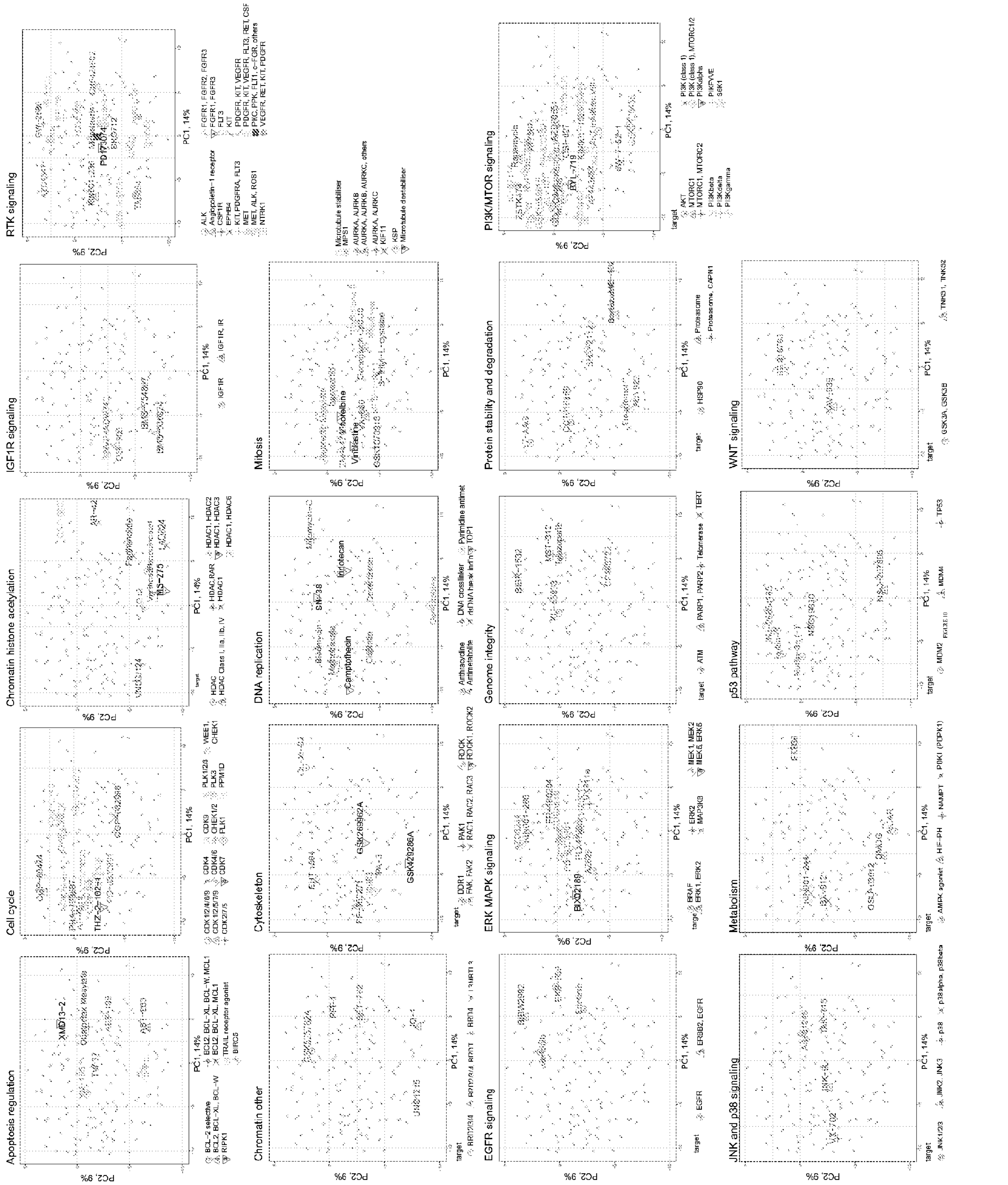
RTK signaling
IGF1R signaling
Chromatin histone acetylation
Cell cycle
Apoptosis regulation
Mitosis
DNA replication
Cytoskeleton
Chromatin other
PI3K/MTOR signaling
Protein stability and degradation
Genome integrity
ERK MAPK signaling
EGFR signaling
WNT signaling
p53 pathway
Metabolism
JNK and p38 signaling

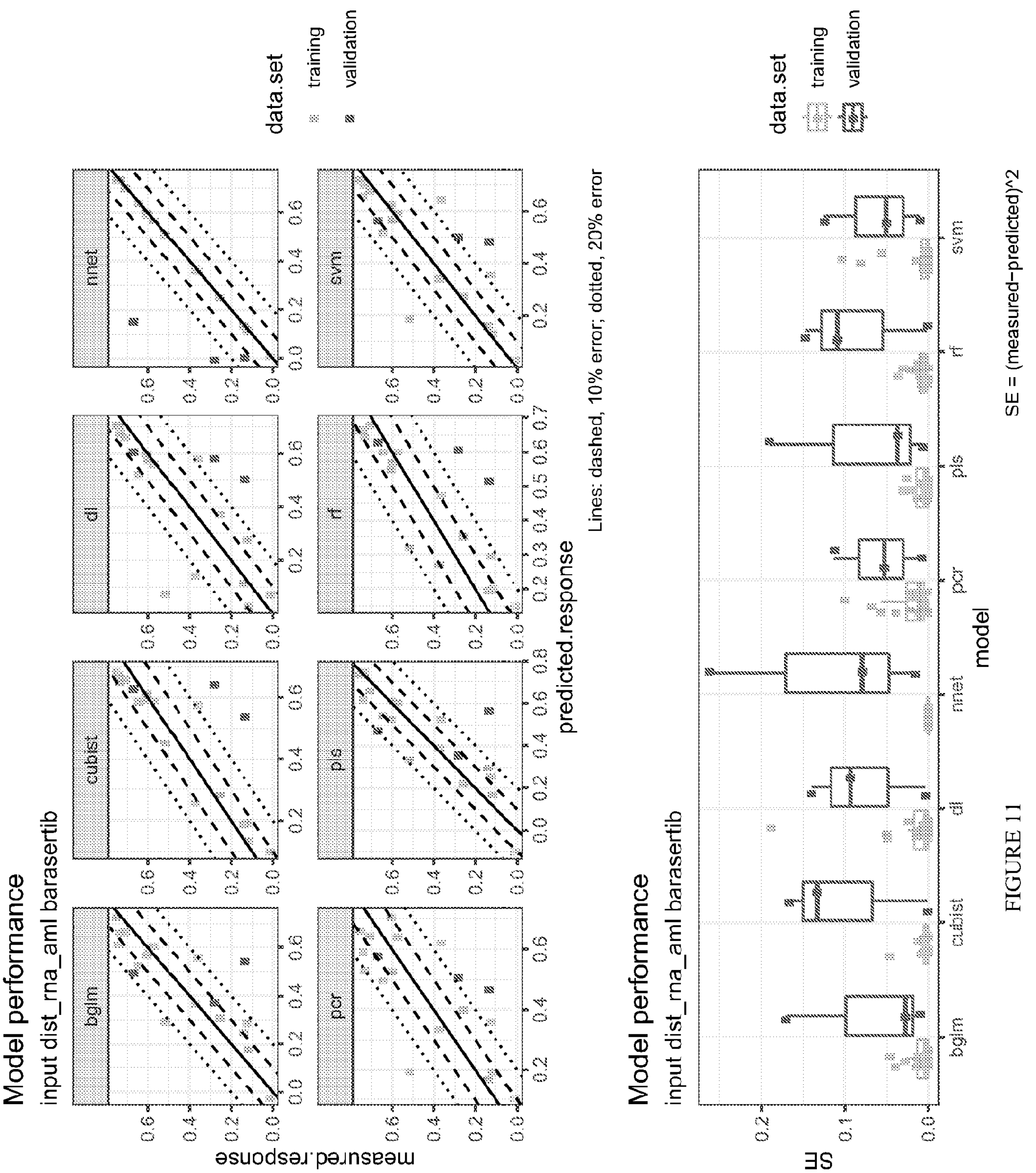
Model performance
input dist_rna_aml barasertib
bglm
cubist
dl
nnet
pcr
pls
rf
svm
measured.response
predicted.response
Lines: dashed, 10% error; dotted, 20% error
data.set
training
validation
Model performance
input dist_rna_aml barasertib
SE
model
bglm
cubist
dl
nnet
pcr
pls
rf
svm
SE = (measured−predicted)^2
data.set
training
validation

FIGURE 11

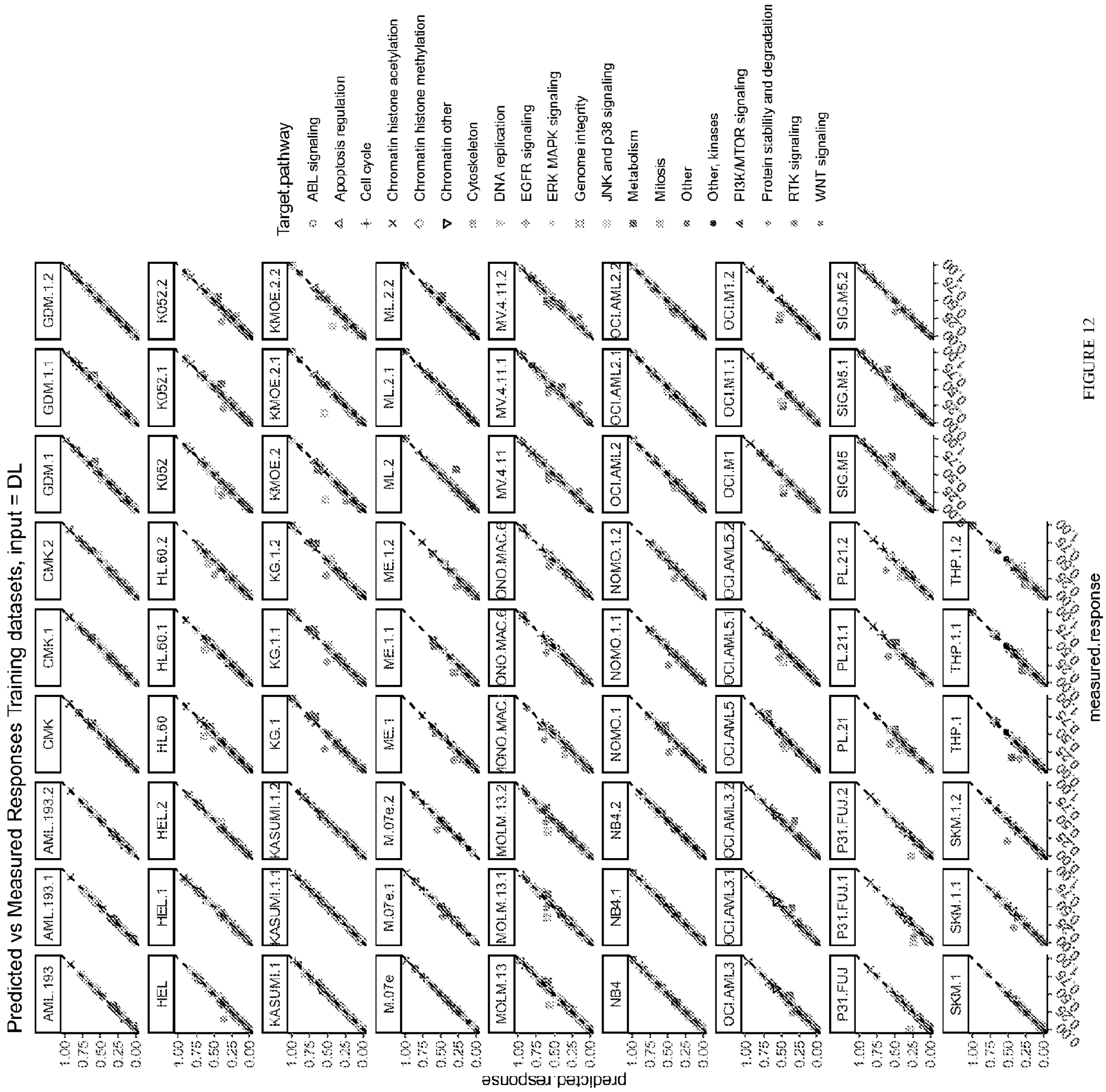
Predicted vs Measured Responses Training datasets, input = DL
predicted.response
measured.response
1.00
0.75
0.50
0.25
0.00
AML.193
AML.193.1
AML.193.2
CMK
CMK.1
CMK.2
GDM.1
GDM.1.1
GDM.1.2
HEL
HEL.1
HEL.2
HL.60
HL.60.1
HL.60.2
K052
K052.1
K052.2
KASUMI.1
KASUMI.1.1
KASUMI.1.2
KG.1
KG.1.1
KG.1.2
KMOE.2
KMOE.2.1
KMOE.2.2
M.07e
M.07e.1
M.07e.2
ME.1
ME.1.1
ME.1.2
ML.2
ML.2.1
ML.2.2
MOLM.13
MOLM.13.1
MOLM.13.2
MV.4.11
MV.4.11.1
MV.4.11.2
NB4
NB4.1
NB4.2
NOMO.1
NOMO.1.1
NOMO.1.2
OCI.AML2
OCI.AML2.1
OCI.AML2.2
OCI.AML3
OCI.AML3.1
OCI.AML3.2
OCI.AML5
OCI.AML5.1
OCI.AML5.2
OCI.M1
OCI.M1.1
OCI.M1.2
P31.FUJ
P31.FUJ.1
P31.FUJ.2
PL.21
PL.21.1
PL.21.2
SIG.M5
SIG.M5.1
SIG.M5.2
SKM.1
SKM.1.1
SKM.1.2
THP.1
THP.1.1
THP.1.2
Target.pathway
ABL signaling
Apoptosis regulation
Cell cycle
Chromatin histone acetylation
Chromatin histone methylation
Chromatin other
Cytoskeleton
DNA replication
EGFR signaling
ERK MAPK signaling
Genome integrity
JNK and p38 signaling
Metabolism
Mitosis
Other
Other, kinases
PI3K/MTOR signaling
Protein stability and degradation
RTK signaling
WNT signaling

FIGURE 12

A Identified peptides in CRC cell lines

| parameter | value |
|---|---|
| Number of peptides | 17538 |
| Unique peptides | 13257 |
| Total phosphopeptides | 16314 |
| Unique phosphopeptides | 12197 |
| Unique S/T peptides | 11590 |
| Unique Y peptides | 1216 |
| Unique Proteins | 3350 |
| Unique Phosphoproteins | 3084 |
| Mascot scr (no mod)* | 50 +/− 24 |
| Mascot scr (phospho)* | 50 +/− 24 |

* *Average +/− SD*

B

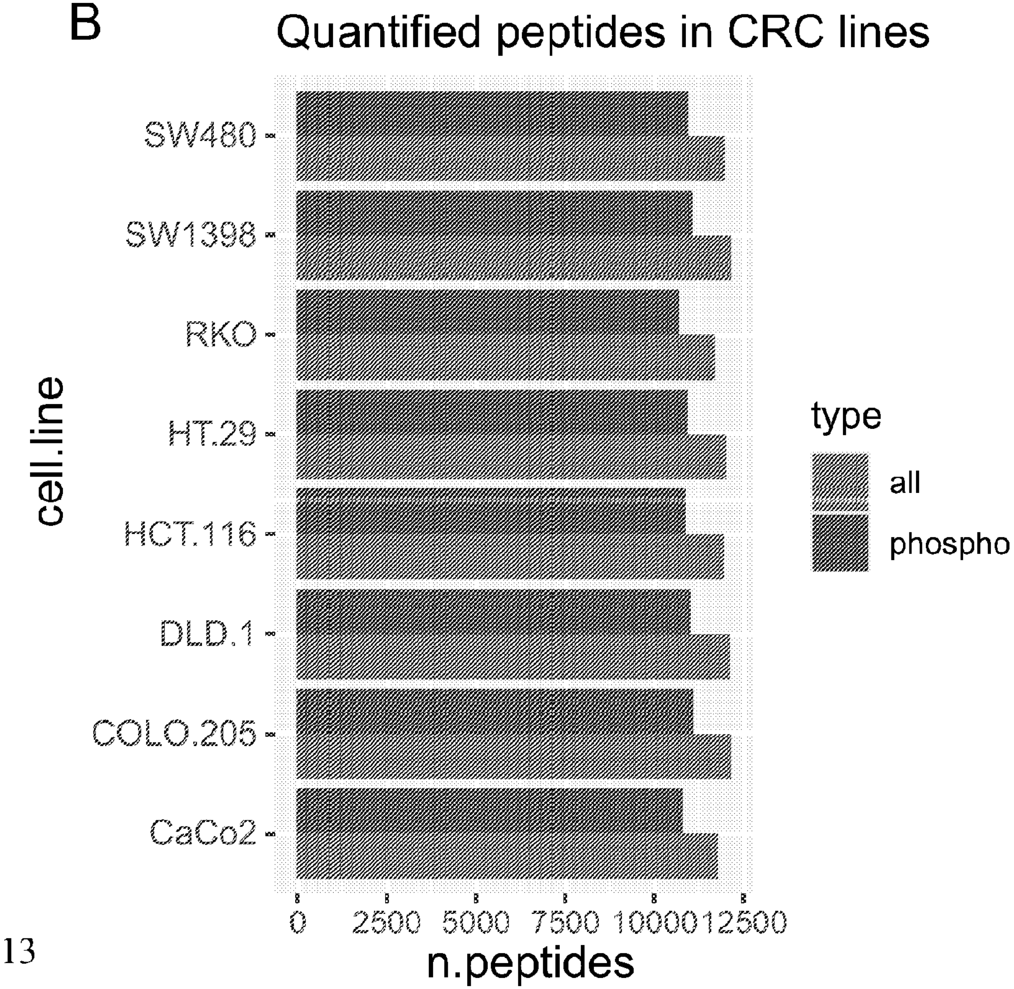


FIGURE 13

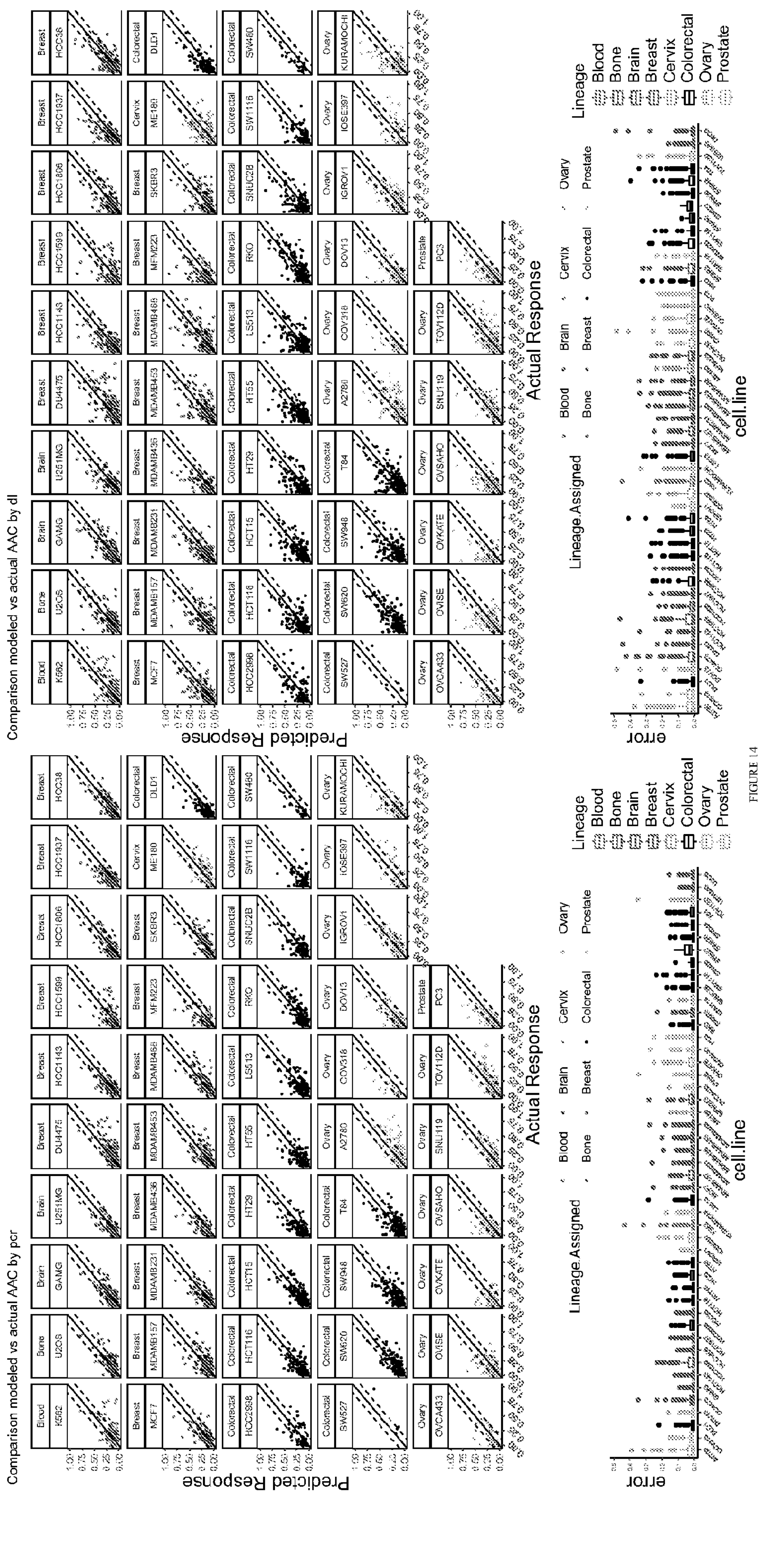
Comparison modeled vs actual AAC by pcr
Comparison modeled vs actual AAC by dl
Predicted Response
Actual Response
error
cell.line
Lineage.Assigned
Blood
Bone
Brain
Breast
Cervix
Colorectal
Ovary
Prostate
Lineage
Blood
Bone
Brain
Breast
Cervix
Colorectal
Ovary
Prostate

FIGURE 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2018190381 A1
- WO 2010119261 A
- GB 2010000770 W
- EP 2016077845 W
- WO 2017085116 A


### Non-patent literature cited in the description


- **MAXQUANT**. *Nature Biotechnology*, 2008, vol. 26, 1367-1372
- **AASEBO, E ; BERVEN, F.S ; BARTAULA-BREVIK, S ; STOKOWY, T ; HOVLAND, R ; VAUDEL, M ; DOSKELAND, S.O ; MCCORMACK, E. ; BATTH, T.S ; OLSEN, J.V et al.** Proteome and Phosphoproteome Changes Associated with Prognosis in Acute Myeloid Leukemia. *Cancers (Basel)*, 2020, vol. 12
- **AEBERSOLD, R ; MANN, M.** Mass-spectrometric exploration of proteome structure and function.. *Nature*, 2016, vol. 537, 347-355
- **BASU, A. ; BODYCOMBE, N.E ; CHEAH, J.H ; PRICE, E.V ; LIU, K ; SCHAEFER, G.I ; EBRIGHT, R.Y ; STEWART, M.L ; ITO, D. ; WANG, S et al.** An interactive resource to identify cancer genetic and lineage dependencies targeted by small molecules.. *Cell*, 2013, vol. 154, 1151-1161
- **BODENMILLER, B. ; WANKA, S ; KRAFT, C. ; URBAN, J ; CAMPBELL, D ; PEDRIOLI, P.G ; GERRITS, B ; PICOTTI, P ; LAM, H ; VITEK, O. et al.** Phosphoproteomic analysis reveals interconnected system-wide responses to perturbations of kinases and phosphatases in yeast. *Science signaling*, 2010, vol. 3, rs4
- **CASADO, P. ; ALCOLEA, M.P ; LORIO, F ; RODRIGUEZ-PRADOS, J.C ; VANHAESEBROECK, B ; SAEZ-RODRIGUEZ, J. ; JOEL, S. ; CUTILLAS, P.R.** Phosphoproteomics data classify hematological cancer cell lines according to tumor type and sensitivity to kinase inhibitors. *Genome biology*, 2013, vol. 14, R37
- **CASADO, P ; RODRIGUEZ-PRADOS, J.C. ; COSULICH, S.C ; GUICHARD, S ; VANHAESEBROECK, B ; JOEL, S ; CUTILLAS, P.R.** Kinase-substrate enrichment analysis provides insights into the heterogeneity of signaling pathway activation in leukemia cells. *Sci Signal*, 2013, vol. 6, rs6
- **CASADO, P ; WILKES, E.H ; MIRAKI-MOUD, F ; HADI, M.M ; RIO-MACHIN, A ; RAJEEVE, V ; PIKE, R ; IQBAL, S ; MARFA, S ; LEA, N et al.** Proteomic and genomic integration identifies kinase and differentiation determinants of kinase inhibitor sensitivity in leukemia cells. *Leukemia*, 2018, vol. 32, 1818-1822
- **CHIU, Y.C ; CHEN, H.H ; ZHANG, T ; ZHANG, S ; GORTHI, A. ; WANG, L.J ; HUANG, Y ; CHEN, Y.** Predicting drug response of tumors from integrated genomic profiles by deep neural networks.. *BMC Med Genomics*, 2019, vol. 12, 18
- **CORSELLO, S.M ; NAGARI, R.T ; SPANGLER, R.D ; ROSSEN, J ; KOCAK, M ; BRYAN, J.G ; HUMEIDI, R ; PECK, D ; WU, X ; TANG, A.A et al.** Discovering the anticancer potential of non-oncology drugs by systematic viability profiling.. *Nature Cancer*, 2020, vol. 1, 235-248
- **CUTILLAS, P.R**. Targeted In-Depth Quantification of Signaling Using Label-Free Mass Spectrometry. *Methods Enzymol*, 2017, vol. 585, 245-268
- **FREJNO, M. ; ZENEZINI CHIOZZI, R ; WILHELM, M ; KOCH, H ; ZHENG, R ; KLAEGER, S ; RUPRECHT, B ; MENG, C ; KRAMER, K ; JARZAB, A et al.** Pharmacoproteomic characterisation of human colon and rectal cancer. *Mol Syst Biol*, 2017, vol. 13, 951
- **HAASE, J. ; BONNER, M.K ; HALAS, H ; KELLY, A.E**. Distinct Roles of the Chromosomal Passenger Complex in the Detection of and Response to Errors in Kinetochore-Microtubule Attachment.. *Dev Cell*, 2017, vol. 42, 640-654, 645
- **HIJAZI, M. ; SMITH, R ; RAJEEVE, V ; BESSANT, C ; CUTILLAS, P.R**. Reconstructing kinase network topologies from phosphoproteomics data reveals cancer-associated rewiring.. *Nat Biotechnol*, 2020, vol. 38, 493-502
- **JARNUCZAK, A.F. ; NAJGEBAUER, H ; BARZINE, M ; KUNDU, D.J ; GHAVIDEL, F ; PEREZ-RIVEROL, Y. ; PAPATHEODOROU, I. ; BRAZMA, A ; VIZCAÍNO, J.A.** An integrated landscape of protein expression in human cancer. *bioRxiv*, 2019, 665968

- **KLEMPNER, S.J.** ; **MYERS, A.P** ; **CANTLEY, L.C.** What a tangled web we weave: emerging resistance mechanisms to inhibition of the phosphoinositide 3-kinase pathway. *Cancer Discov*, 2013, vol. 3, 1345-1354
- **LECUN, Y** ; **BENGIO, Y** ; **HINTON, G**. Deep learning. *Nature*, 2015, vol. 521, 436-444
- **LEUTERT, M** ; **RODRIGUEZ-MIAS, R.A.** ; **FUKUDA, N.K** ; **VILLEN, J**. R2-P2 rapid-robotic phosphoproteomics enables multidimensional cell signaling studies. *Mol Syst Biol*, 2019, vol. 15, e9021
- **MENDEN, M.P** ; **WANG, D** ; **MASON, M.J** ; **SZALAI, B** ; **BULUSU, K.C** ; **GUAN, Y** ; **YU, T** ; **KANG, J** ; **JEON, M.** ; **WOLFINGER, R. et al.** Community assessment to advance computational prediction of cancer drug combinations in a pharmacogenomic screen. *Nat Commun*, 2019, vol. 10, 2674
- **MONTOYA, A** ; **BELTRAN, L.** ; **CASADO, P** ; **RODRIGUEZ-PRADOS, J.C** ; **CUTILLAS, P.R.** Characterization of a TiO(2) enrichment method for label-free quantitative phosphoproteomics.. *Methods (San Diego, Calif)*, 2011, vol. 54, 370-378
- **PAULITSCHKE, V** ; **EICHHOFF, O** ; **GERNER, C** ; **PAULITSCHKE, P** ; **BILECK, A** ; **MOHR, T** ; **CHENG, P.F** ; **LEITNER, A** ; **GUENOVA, E** ; **SAULITE, I et al.** Proteomic identification of a marker signature for MAPKi resistance in melanoma.. *EMBO J*, 2019, vol. 38, e95874
- **PIERSMA, S.R.** ; **KNOL, J.C** ; **DE REUS, I.** ; **LABOTS, M** ; **SAMPADI, B.K** ; **PHAM, T.V** ; **ISHIHAMA, Y** ; **VERHEUL, H.M** ; **JIMENEZ, C.R**. Feasibility of label-free phosphoproteomics and application to base-line signaling of colorectal cancer cell lines. *Journal of proteomics*, 2015, vol. 127, 247-258
- **ROUMELIOTIS, T.I.** ; **WILLIAMS, S.P.** ; **GONCALVES, E** ; **ALSINET, C** ; **DEL CASTILLO VELASCO-HERRERA, M** ; **ABEN, N** ; **GHAVIDEL, F.Z.** ; **MICHAUT, M** ; **SCHUBERT, M** ; **PRICE, S et al.** Genomic Determinants of Protein Abundance Variation in Colorectal Cancer Cells. *Cell Rep*, 2017, vol. 20, 2201-2214
- **RUDOLPH, J.D** ; **DE GRAAUW, M** ; **VAN DE WATER, B** ; **GEIGER, T** ; **SHARAN, R.** Elucidation of Signaling Pathways from Large-Scale Phosphoproteomic Data Using Protein Interaction Networks. *Cell Syst*, 2016, vol. 3, 585-593, 583
- **SAKELLAROPOULOS, T.** ; **VOUGAS, K** ; **NARANG, S** ; **KOINIS, F** ; **KOTSINAS, A** ; **POLYZOS, A** ; **MOSS, T.J** ; **PIHA-PAUL, S** ; **ZHOU, H.** ; **KARDALA, E et al.** A Deep Learning Framework for Predicting Response to Therapy in Cancer. *Cell Rep*, 2019, vol. 29, 3367-3373, 3364
- **SMIRNOV, P** ; **KOFIA, V** ; **MARU, A** ; **FREEMAN, M** ; **HO, C** ; **EL-HACHEM, N** ; **ADAM, G.A** ; **BA-ALAWI, W** ; **SAFIKHANI, Z.** ; **HAIBE-KAINS, B.** PharmacoDB: an integrative database for mining in vitro anticancer drug screening studies.. *Nucleic Acids Res*, 2018, vol. 46, D994-D1002
- **VAN ALPHEN, C** ; **CLOOS, J.** ; **BEEKHOF, R** ; **CUCCHI, D.G.J** ; **PIERSMA, S.R** ; **KNOL, J.C** ; **HENNEMAN, A.A** ; **PHAM, T.V.** ; **VAN MEERLOO, J** ; **OSSENKOPPELE, G.J et al.** Phosphotyrosine-based Phosphoproteomics for Target Identification and Drug Response Prediction in AML Cell Lines.. *Mol Cell Proteomics*, 2020, vol. 19, 884-899
- **VOWINCKEL, J** ; **ZELEZNIAK, A** ; **BRUDERER, R** ; **MULLEDER, M** ; **REITER, L** ; **RALSER, M**. Cost-effective generation of precise label-free quantitative proteomes in high-throughput by microLC and data-independent acquisition. *Sci Rep*, 2018, vol. 8, 4346
- **WILKES, E.** ; **CUTILLAS, P.R.** Label-Free Phosphoproteomic Approach for Kinase Signaling Analysis. *Methods Mol Biol*, 2017, vol. 1636, 199-217
- **WILKES, E.H** ; **TERFVE, C** ; **GRIBBEN, J.G.** ; **SAEZ-RODRIGUEZ, J** ; **CUTILLAS, P.R.** Empirical inference of circuitry and plasticity in a kinase signaling network.. *Proc Natl Acad Sci U S A*, 2015, vol. 112, 7719-7724
- **YANG, W.** ; **SOARES, J** ; **GRENINGER, P** ; **EDELMAN, E.J** ; **LIGHTFOOT, H** ; **FORBES, S** ; **BINDAL, N** ; **BEARE, D** ; **SMITH, J.A** ; **THOMPSON, I.R. et al.** Genomics of Drug Sensitivity in Cancer (GDSC): a resource for therapeutic biomarker discovery in cancer cells. *Nucleic Acids Res*, 2013, vol. 41, D955-961